# EUROPEAN PATENT SPECIFICATION

(11) **EP 3 909 591 B1**
(45) Date of publication and mention of the grant of the patent: **24.07.2024**
(21) Application number: 19897505.4
(22) Date of filing: 04.12.2019
(51) Int. Cl.: A61K 35/28, A61P 1/18, C12N 5/0775

(54) **PHARMACEUTICAL COMPOSITION FOR TREATING PANCREATITIS, COMPRISING CLONAL STEM CELLS**
PHARMAZEUTISCHE ZUSAMMENSETZUNG ZUR BEHANDLUNG VON PANKREATITIS MIT KLONALEN STAMMZELLEN
COMPOSITION PHARMACEUTIQUE POUR LE TRAITEMENT DE LA PANCRÉATITE, COMPRENANT DES CELLULES SOUCHES CLONALES

(30) Priority: 13.12.2018 KR 20180160667
(43) Date of publication of application: 17.11.2021
(73) Proprietor: SCM Lifescience Co., Ltd., Jung-gu Incheon 22332 (KR)
(72) Inventor: SONG, Sun Uk, Incheon 22003 (KR); KIM, Si Na, Incheon 21997 (KR); HWANG, Myeong Hwan, Bucheon-si Gyeonggi-do 14782 (KR)
(74) Representative: Altmann Stößel Dick Patentanwälte PartG mbB
(86) International application number: PCT/KR2019/017029
(87) International publication number: WO 2020/122498

(56) References cited:
- WO-A2-2008/002914
- KR-A- 20100 120 532
- KR-A- 20130 106 432
- KR-A- 20170 111 057
- KR-B1- 100 802 011
- KAWAKUBO KAZUMICHI ET AL: "Mesenchymal stem cell therapy for acute and chronic pancreatitis", JOURNAL OF GASTROENTERLOGY, SPRINGER JAPAN KK, JP, vol. 53, no. 1, 23 June 2017 (2017-06-23), pages 1 - 5, XP036388662, ISSN: 0944-1174, [retrieved on 20170623], DOI: 10.1007/S00535-017-1363-9
- JUNG KYUNG HEE ET AL: "Therapeutic effect of human clonal bone marrow-derived mesenchymal stem cells in severe acute pancreatitis", ARCHIVES OF PHARMACAL RESEARCH, NATL. FISHERIES UNIVERSITY , PUSAN, KR, vol. 38, no. 5, 21 August 2014 (2014-08-21), pages 742 - 751, XP035501365, ISSN: 0253-6269, [retrieved on 20140821], DOI: 10.1007/S12272-014-0465-7
- JUNG, K. H. ET AL.: "Human bone marrow-derived clonal mesenchymal stem cells inhibit inflammation and reduce acute pancreatitis in rats", GASTROENTEROLOGY, vol. 140, March 2011 (2011-03-01), pages 998 - 1008 .e4, XP028253803
- YI, T. ET AL.: "Manufacture of clinical-grade human clonal mesenchymal stem cell products from single colony forming unit-derived colonies based on the subfractionation culturing method", TISSUE ENGINEERING PART C, vol. 21, no. 12, 12 November 2015 (2015-11-12), pages 1251 - 1262, XP009509021, DOI: 10.1089/ten.tec.2015.0017

## Description

### [Technical Field]

The present disclosure relates to a pharmaceutical composition for use in preventing or treating, pancreatitis, the pharmaceutical composition comprising monoclonal stem cells obtained by improved subfractionation culture of said monocolonal stem cells, and to a method of producing a pharmaceutical composition comprising monoclonal stem cells.

### [Background Art]

Pancreatitis is a disease developed by the inflammation in the pancreas, which is divided into acute pancreatitis and chronic pancreatitis. Pancreatitis is developed when autolysis of the pancreas is induced by the enzymes contained in pancreatic juice because the pancreatic juice does not flow smoothly due to alcohol abuse and gallstones, etc. There are two kinds of pancreatitis in general, which are mild type pancreatitis accompanied by interstitial edema and peripancreatic fat necrosis around the pancreas; and severe type pancreatitis accompanied by broad peripancreatic and intrapancreatic fat necrosis, pancreatic parenchymal necrosis, and hemorrhage.

The exact pathophysiological mechanism of pancreatitis has not been disclosed but is believed to be an autolysis process caused by the early activation of protease precursors in the pancreas appearing as a representative symptom. In other words, once a digestive enzyme is abnormally prematurely activated in pancreas acinar cells, the pancreatic acinus itself is digested and accordingly the inflammation occurs to cause the separation and death of the pancreatic tissues. It has recently been reported that the activated macrophages infiltrating into pancreas after injury of pancreas acinar cells induce the secretion of the proinflammatory cytokine interleukin-1β as a response to the tissue damage, suggesting that the macrophages play an important role in circulation of inflammatory cells, pancreatic edema, and actual pancreas destruction.

Several experimental treatment methods have been proposed to alleviate the severity of pancreatitis and inhibit the development of complications in various organs. However, when these experimental treatment methods were applied to patients, the effect was not so great, so that there are no effectively widely used drugs so far in relation to the prevention and treatment of pancreatitis.

Recently, attempts are being made to use stem cells for the treatment of various diseases. Stem cells have potential to grow into tissues of all 210 organs in our body, which can be infinitely divided and can be differentiated into desired organs by the suitable manipulation. Due to such characteristics, stem cells are in the spotlight as a new therapeutic agent. The treatability of incurable diseases using stem cells is very high, and thus it is expected to be able to treat numerous diseases such as leukemia, osteoporosis, hepatitis, Parkinson's disease, senile dementia, and burns.

However, stem cells still have many limitations in that it is difficult to obtain stem cells in large quantities. Although the method of obtaining stem cells from frozen embryonic cells may be effective, there is still a lot of controversy in terms of ethics. In order to address these issues, many studies have also been conducted on a method of obtaining stem cells using adult stem cells or a somatic cell nucleus transplantation method. The field that is more actively conducted than research on embryonic stem cells is adult stem cell research. Adult stem cells are cells that remain in various organs such as the central nervous system or bone marrow and participate in the organ development during the growth period and the regeneration of damage. Since adult stem cells are present in various organs, they can be obtained from various sites including bone marrow, spleen, adipocytes, and the like, but the method obtained from bone marrow is most commonly practiced. However, it is difficult to obtain uniformly shaped cells at all times while mesenchymal stem cells among many kinds of bone marrow cells are isolated and cultured. Thus, studies are conducted to address these issues.

WO2012/092603 discloses a method of treating pancreatitis by administering to the patient mesenchymal stem cells that are obtained by manipulating a biological sample of cells, which includes multi-lineage stem cells, progenitor cells, other marrow stromal cells: allowing the sample of cells to settle in a container; transferring supernatant from the container to another container; and isolating cells from the supernatant, which has comparatively lower density in the sample.

KR 2010 0120 532 discloses a method for reactivating multipotency and proliferation rate of aged adult stem cells.

WO 2008/002914 discloses a method of expanding mesenchymal stem cells ex vivo including the steps of seeding a collection of cells containing the mesenchymal stem cells on a substrate so that a low density of mesenchymal stem cells adhere to the substrate; expanding the adhered mesenchymal stem cells on the substrate; removing the expanded mesenchymal stem cells from the substrate; and reseeding the mesenchymal stem cells on the same or different substrate.

The present inventors have invented a method for isolating stem cells named as a novel subfractionation culturing method, and filed a patent application under Korean Patent Application No. 10-2006-0075676, which was granted for patent. The subfractionation culturing method may be performed at a lower cost than other methods. Moreover, there is no contamination issue, and clonal mesenchymal stem cells (cMSCs) can be obtained effectively without risk to mix other stem cells. Therefore, the subfractionation culturing method shows the unexpected superiority compared to the other methods of obtaining stem cells. However, despite the superiority of the method, the subfractionation culturing method has limitations in which it is difficult to rapidly obtain the monoclonal mesenchymal stem cell group. These are because in order to mass-produce mesenchymal stem cells to be used as a final product, the method requires production of a working cell bank which is used for carrying out the process of obtaining the final product, thereby obtaining a sufficient amount of mesenchymal stem cells and needs the culture with at least passage 10.

The use of stem cells to treat inflammatory diseases, particularly pancreatitis, still has various limitations, and there is no known stem cell preparation method for effectively treating pancreatitis and a method of treating pancreatitis using the same.

### [Disclosure]

### [Technical Problem]

While conducting research to induce rapid proliferation of stem cells through the improvement of the subfractionation culturing method as described above, the present inventors identified that when an improved subfractionation culturing method in which culture cell density is adjusted low and an antioxidant is added was used, it was possible to induce an increase in the effective cell proliferation rate only with a few subcultures, and that the monoclonal stem cells obtained therethrough exhibited a very remarkable effect of treating pancreatitis compared to the stem cells of the conventional subfractionation culturing method, and then completed the present disclosure.

Accordingly, an aspect of the present disclosure is directed to providing a pharmaceutical composition for use in preventing or treating pancreatitis, the pharmaceutical composition comprising monoclonal stem cells obtained through a method of subfractionation culture and proliferation of said monoclonal stem cells that has improved the conventional subfractionation culturing method, and a method of producing a pharmaceutical composition comprising monoclonal stem cells .

### [Technical Solution]

An exemplary embodiment of the present disclosure provides a pharmaceutical composition for use in preventing or treating pancreatitis, the pharmaceutical composition comprising monoclonal stem cells obtained by steps of: 1) culturing bone marrow isolated from a subject in a first vessel; 2) transferring only a supernatant from the first vessel to a new vessel and culturing the supernatant; 3) culturing cells present in the new vessel and obtaining a supernatant; 4) obtaining the monoclonal stem cells by repeating steps 2) and 3) at least once using the supernatant of step 3) as the supernatant of the first vessel of step 2); 5) inoculating the monoclonal stem cells of step 4) in a medium at a cell density of 50 to 1,000 cells/cm² to culture the cells at passage 1; and 6) inoculating the subcultured monoclonal stem cells of step 5) in a medium at a cell density of 50 to 1000 cells/cm² to culture the cells at passages 2 to 8, wherein the culture medium in the step 5) and 6) further includes an antioxidant.

In addition, an exemplary embodiment of the present disclosure provides a method of producing a pharmaceutical composition comprising monoclonal stem cells, the method comprising steps of 1) culturing bone marrow isolated from a subject in a first vessel; 2) transferring only a supernatant from the first vessel to a new vessel and culturing the supernatant; 3) culturing cells present in the new vessel and obtaining a supernatant; 4) obtaining monoclonal stem cells by repeating steps 2) and 3) at least once using the supernatant of step 3) as the supernatant of the first vessel of step 2); 5) inoculating the monoclonal stem cells of step 4) in a medium at a cell density of 50 to 1,000 cells/cm² to culture the cells at passage 1; and 6) inoculating the subcultured monoclonal stem cells of step 5) in a medium at a cell density of 50 to 1000 cells/cm² to culture the cells at passages 2 to 8, wherein the culture medium in the step 5) and 6) further includes an antioxidant.

### [Advantageous Effects]

According to an improved method of subfractionation culture and proliferation of stem cells of the present disclosure, it is possible to obtain a large quantity of desired monoclonal stem cells in a short time by rapid proliferation of monoclonal stem cells, and the monoclonal mesenchymal stem cells obtained thereby are stem cells with enhanced therapeutic effects on pancreatitis, and thus may be usefully used as a therapeutic agent for pancreatitis.

### [Description of Drawings]

FIG. 1 is a view showing a subfractionation culturing method for isolating monoclonal mesenchymal stem cells from bone marrow.
FIG. 2 is a view showing the results of identifying the morphological changes in monoclonal mesenchymal stem cells according to cell culture density and cell subculture through microscopic observation.
FIG. 3 is a view showing the results of identifying the changes in cell size and granularity of monoclonal mesenchymal stem cells according to cell culture density and cell subculture by an average value of forward-scattered (FSC) (A) light and side-scattered (SSC) (B) light through flow cytometry (FACS) analysis (*p < 0.05, **p < 0.01, ***p < 0.005).
FIG. 4 is a view showing the results of identifying whether cells are aged after staining monoclonal mesenchymal stem cells by beta galactosidase (beta gal) activity in which cell culture densities and cell culture subcultures vary.
FIG. 5 is a view showing the results of identifying the aging-related genes p15 and p16 and the proliferation marker PCNA by RT-PCR after culturing the monoclonal mesenchymal stem cells of passage 15 (P15) while the cell culture density varies.
FIG. 6 is a view showing the results of identifying the proliferation capacity of monoclonal mesenchymal stem cells through population doubling time (PDT) and population doubling level (PDL) according to cell culture density and cell subculture (*p < 0.05, **p < 0.01, ***p < 0.005).

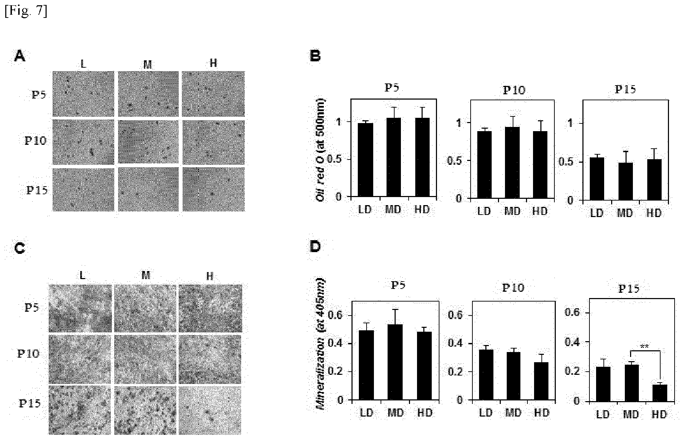

FIG. 7 is a view showing the results of identifying the differentiation ability of monoclonal mesenchymal stem cells according to cell culture density and cell subculture (*p < 0.05, **p < 0.01, ***p < 0.005). FIG. 7A is a result of identifying the differentiation potential of monoclonal mesenchymal stem cells into adipocytes through Oil red O histological staining according to cell culture and cell subculture. FIG. 7B is a view showing quantification of the degree of histological staining of FIG. 7A. FIG. 7C is a result of identifying the differentiation potential of monoclonal mesenchymal stem cells into ossified cells through Alizarin red S histological staining according to cell culture and cell subculture. FIG. 7D is a view showing quantification of the degree of histological staining of FIG. 7C.
FIG. 8 is a view showing the results of total reactive oxygen species (ROS) production of monoclonal mesenchymal stem cells according to cell culture density and cell subculture (A) and identifying DNA damage accordingly through comet assay (B) (*p < 0.05, **p < 0.01, ***p < 0.005).
FIG. 9 is a view showing the results of measuring the concentration of 8-oxo-deoxyguanosine (8-hydroxy-2'-deoxyguanosine; 8-OHdG) in order to identify the degree of DNA damage caused by reactive oxygen species produced according to cell culture density and cell subculture (*p < 0.05, **p < 0.01, ***p < 0.005).
FIG. 10 is a view showing the results of identifying the changes in the proliferation capacity of cells obtained by culturing monoclonal mesenchymal stem cells of passages 11 (P11) to 15 (P15) under the condition of only high-density (HD) or high-density + ascorbic acid (a kind of antioxidant) addition (HD + AA).
FIG. 11 is a view showing the results of comparing the level of reactive oxygen species produced after culturing monoclonal mesenchymal stem cells of passage 15 (P15) under the condition of only high-density (HD) or high-density conditions + ascorbic acid addition (HD + AA) (*p < 0.05, **p < 0.01, ***p < 0.005).
FIG. 12 is a diagram illustrating a comparison of the experimental methods of the conventional subfractionation culturing method and the improved subfractionation culturing method.

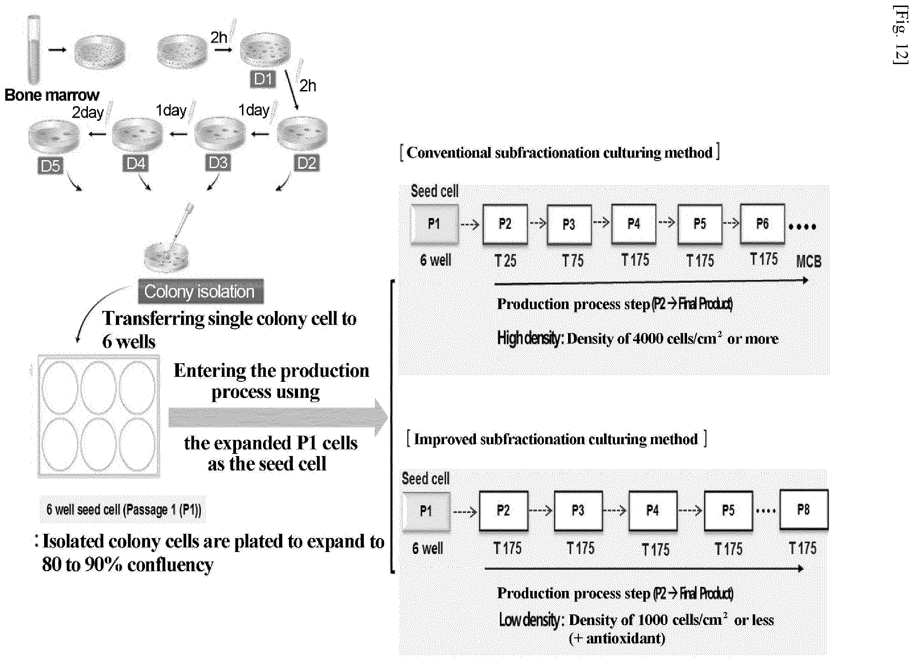

FIG. 12 is a schematic view of an improved subfractionation culturing method, and is a schematic view illustrating a low-density culture corresponding to the culture at passage 2 or later that is different from the conventional subfractionation culturing method.

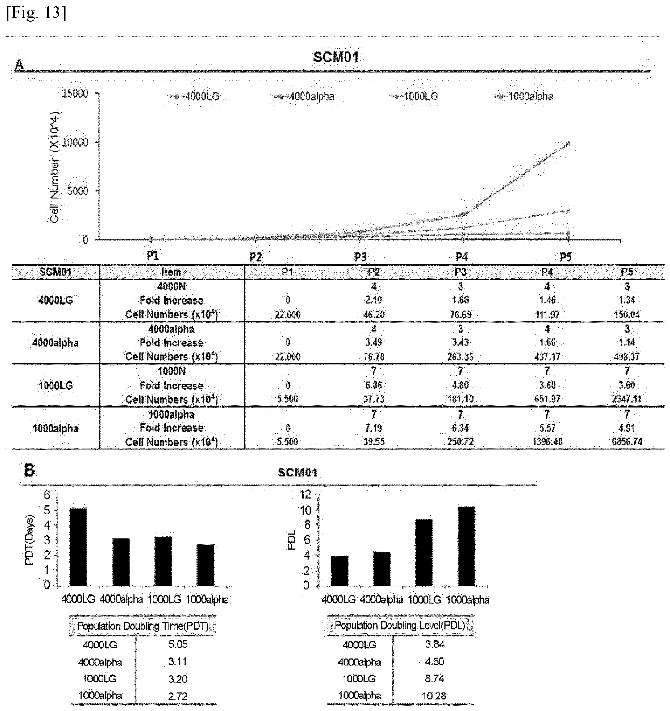

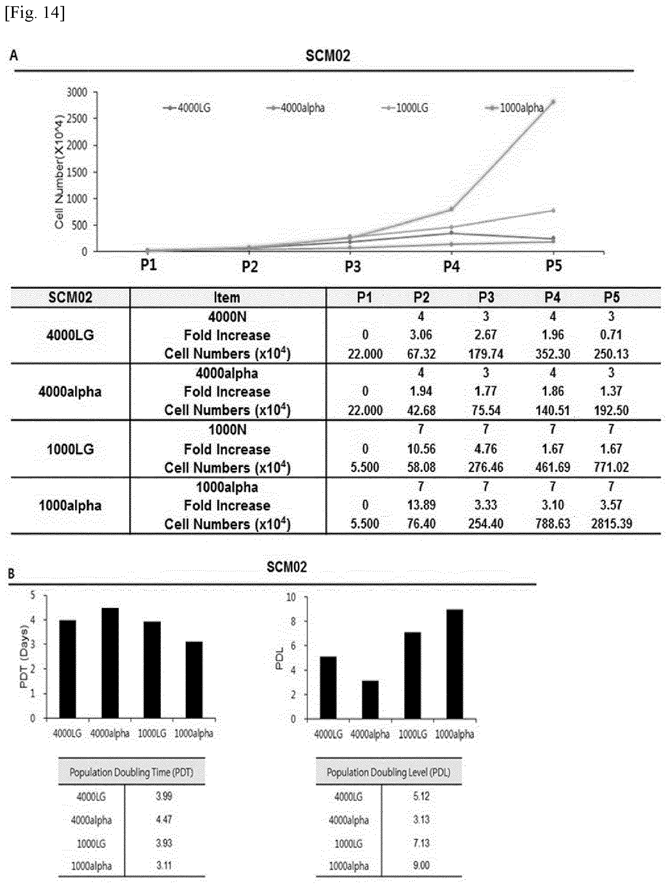

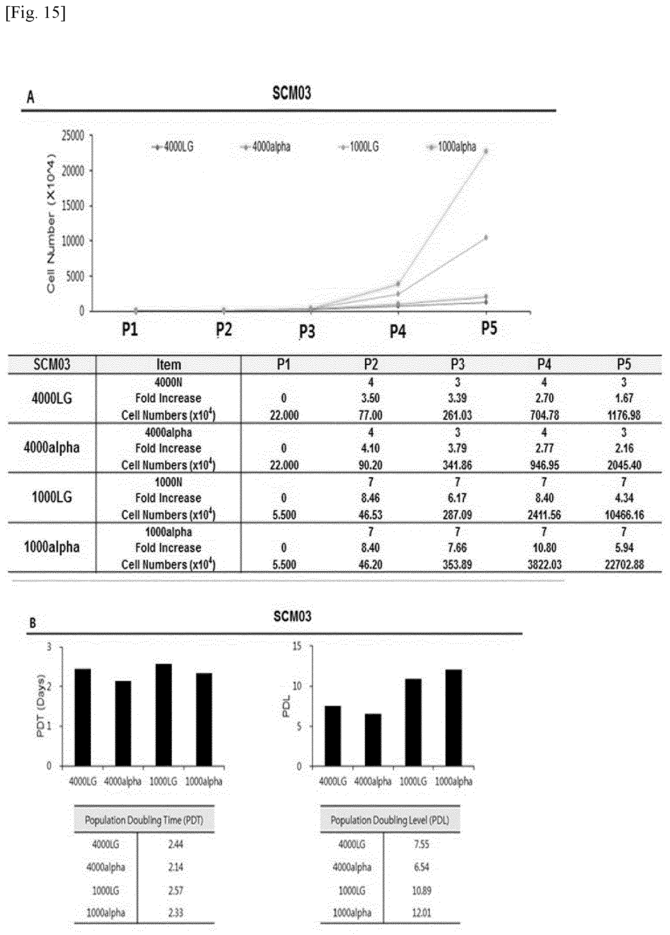

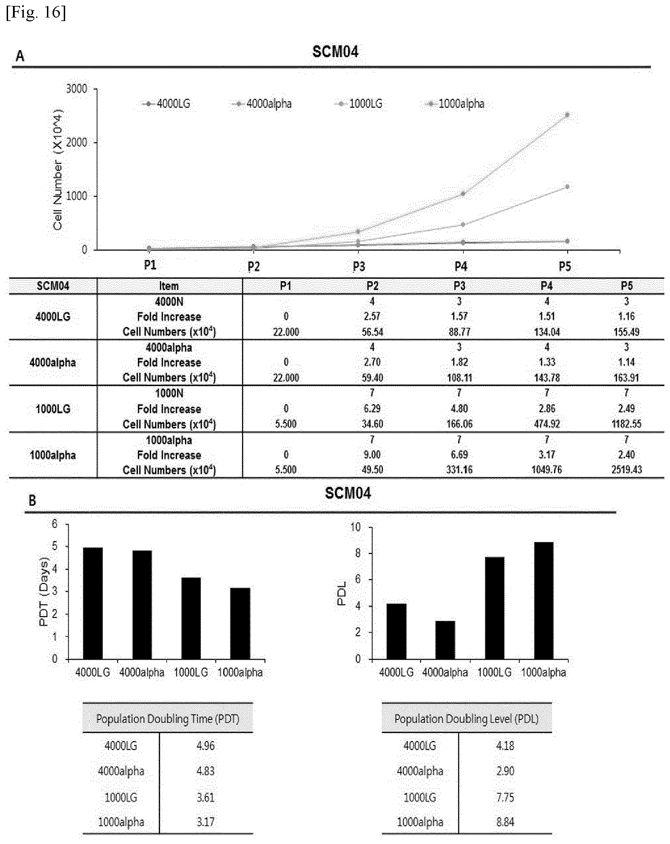

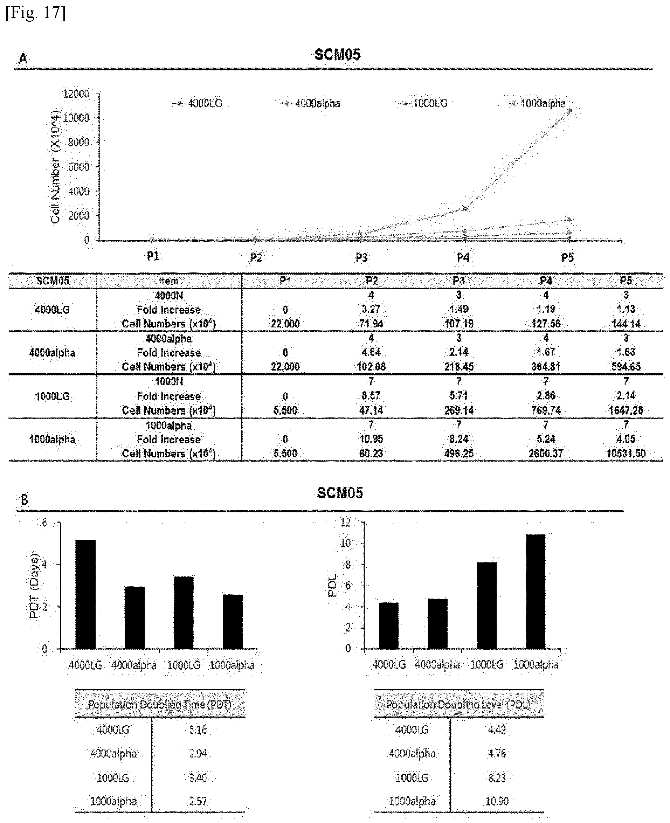

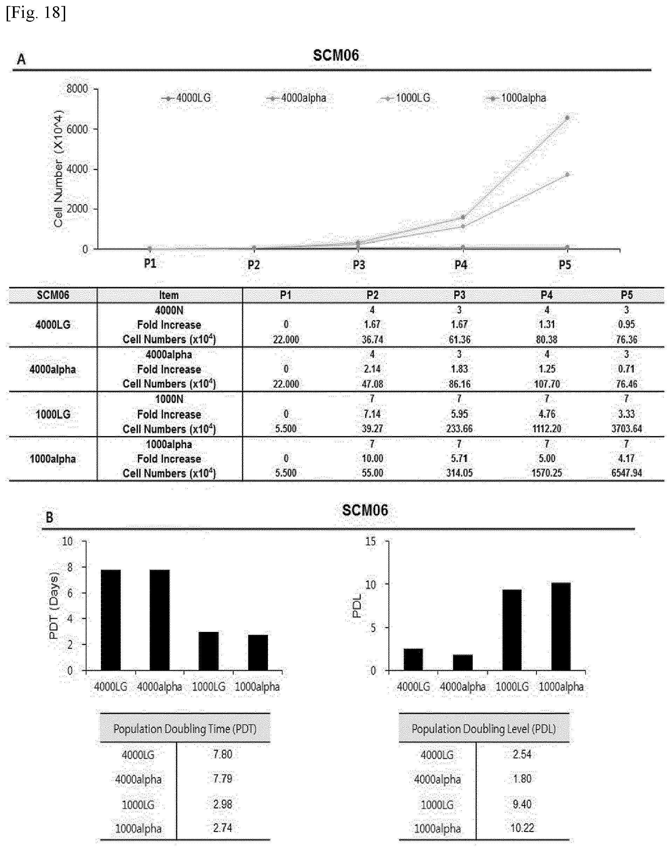

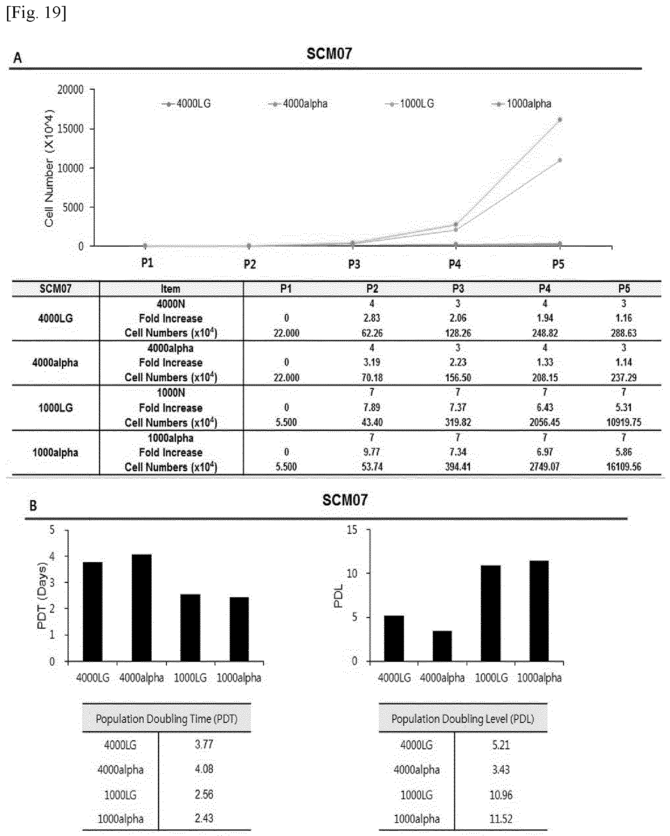

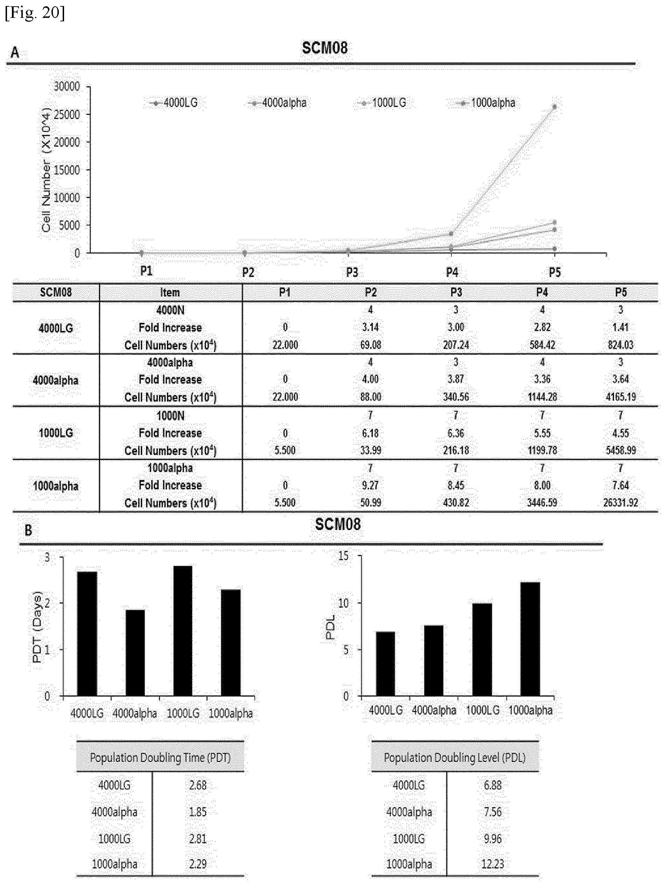

FIGS. 13 to 20 are views showing the results of identifying proliferation rate of cells in which SCM01 to SCM08 monoclonal mesenchymal stem cells obtained by the subfractionation culturing method are inoculated at a density of 1,000 or 4,000 cells/cm², and the cells are cultured using LG-DMEM (Dulbecco's Modified Eagle Medium, low glucose) and α-MEM (Minimum Essential Medium α) culture media with or without an antioxidant. A in each view shows the changes in the number of cells according to passage 1 (P1) to passage 5 (P5) of each experimental group, and B in each diagram shows the results of PDT and PDL of each experimental group.

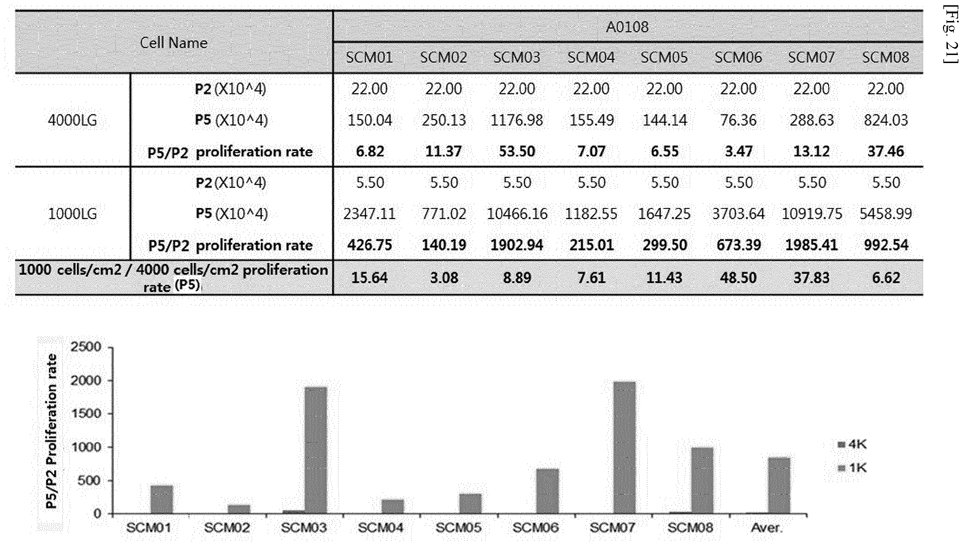

FIG. 21 is a view showing the results of identifying the cell proliferation rate of an experimental group in which a LG-DMEM medium without an antioxidant is used, and only the cell density varies at a density of 1,000 or 4,000 cells/cm².

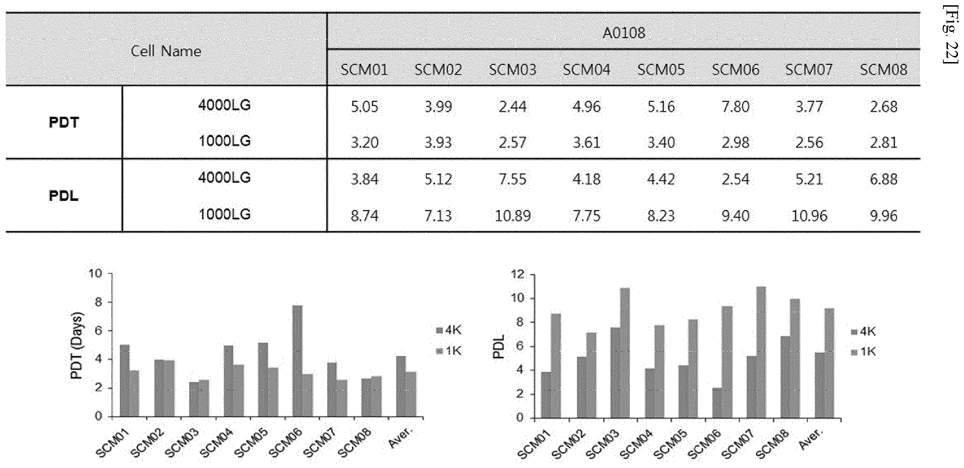

FIG. 22 is a view showing the results of identifying PDT and PDL of an experimental group in which a LG-DMEM medium without an antioxidant is used, and only the cell density varies at a density of 1,000 or 4,000 cells/cm².

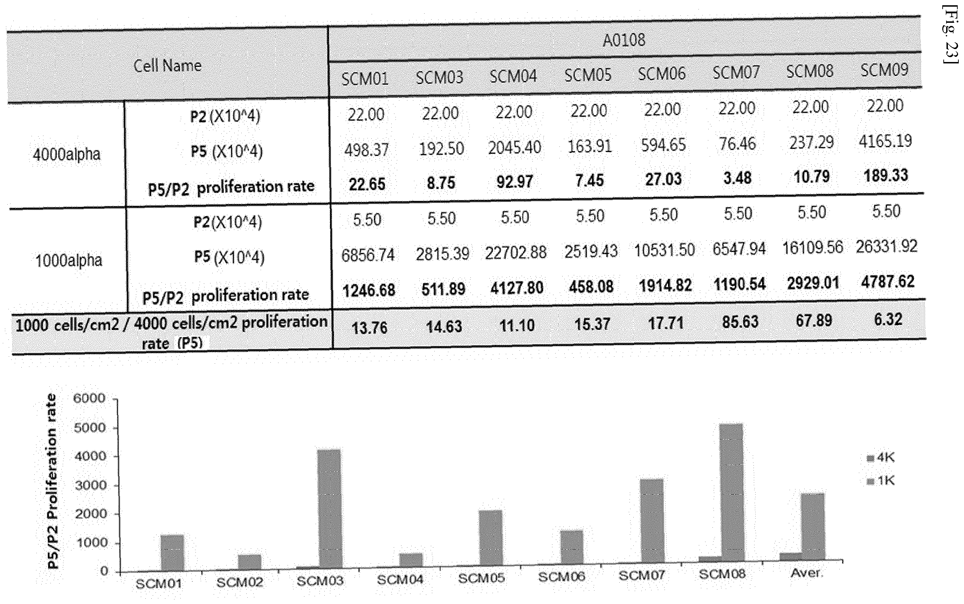

FIG. 23 is a view showing the results of identifying the cell proliferation rate of an experiment group in which a α-DMEM medium with an antioxidant is used, and only the cell density varies at a density of 1,000 or 4,000 cells/cm².

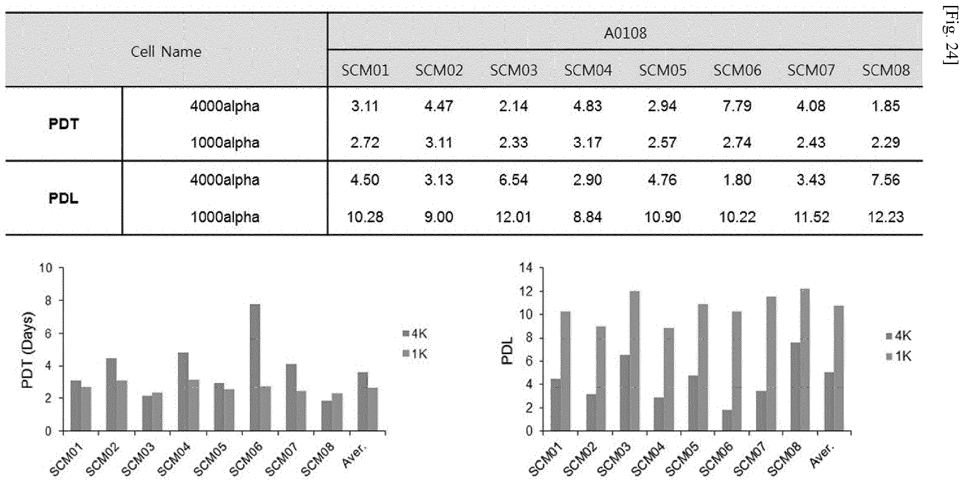

FIG. 24 is a view showing the results of identifying PDT and PDL of an experimental group in which a α-DMEM medium with an antioxidant is used, and only the cell density varies at a density of 1,000 or 4,000 cells/cm².

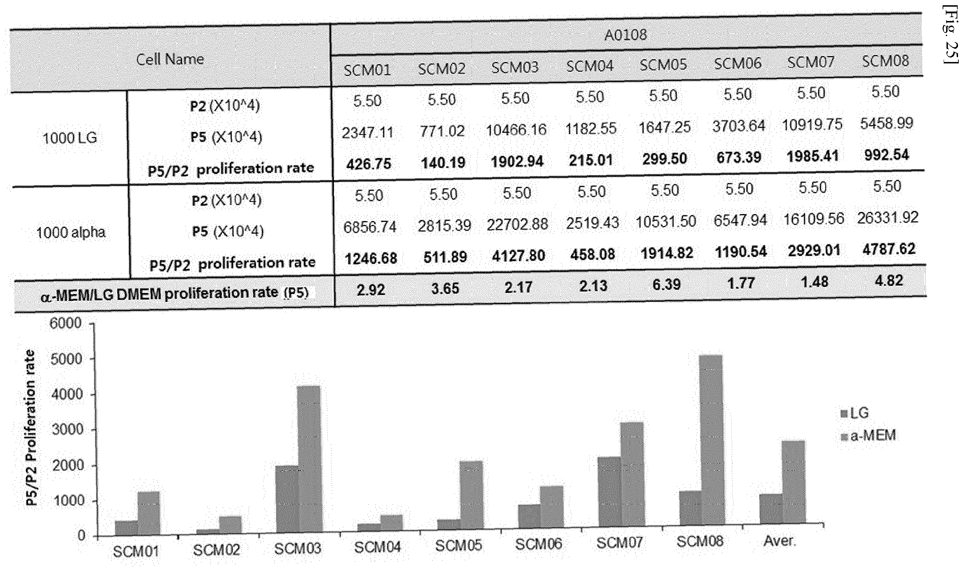

FIG. 25 is a view showing the results of identifying the cell proliferation rate of an experimental group in which the cell density is fixed at a density of 1,000 cells/cm² and the culture medium is LG-DMEM or α-MEM.

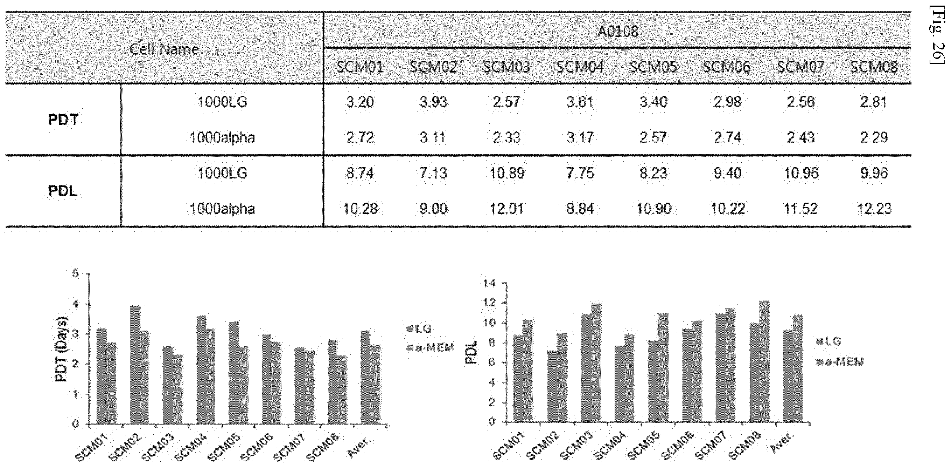

FIG. 26 is a view showing the results of identifying PDT and PDL of an experimental group in which the cell density is fixed at a density of 1,000 cells/cm² and the culture medium is LG-DMEM or α-MEM.

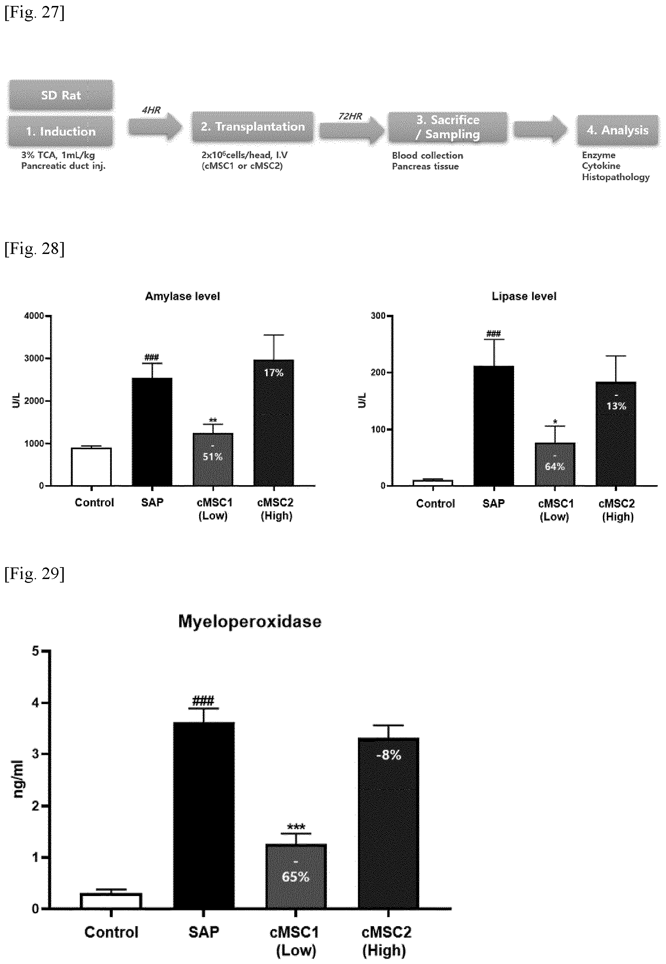

FIG. 27 is a schematic view of acute pancreatitis animal model construction and cMSC1, 2 treatment protocol of the present disclosure.
FIG. 28 is a view showing the results of identifying the changes in the activity of α-amylase and lipase enzymes according to cMSC1 and 2 treatment of the present disclosure in an acute pancreatitis animal model (All values are presented as means with standard error of mean (SEM) P value = *, < 0.05; **, < 0.01; ***, < 0.001 vs. SAP group and ###, < 0.001 vs. control group).
FIG. 29 is a view showing the results of identifying the changes in myeloperoxidase (MPO) activity according to cMSC1 and 2 treatment in an acute pancreatitis animal model (All values are presented as means with standard error of mean (SEM) P value = *, < 0.05; **, < 0.01; ***, < 0.001 vs. SAP group and ###, < 0.001 vs. control group).

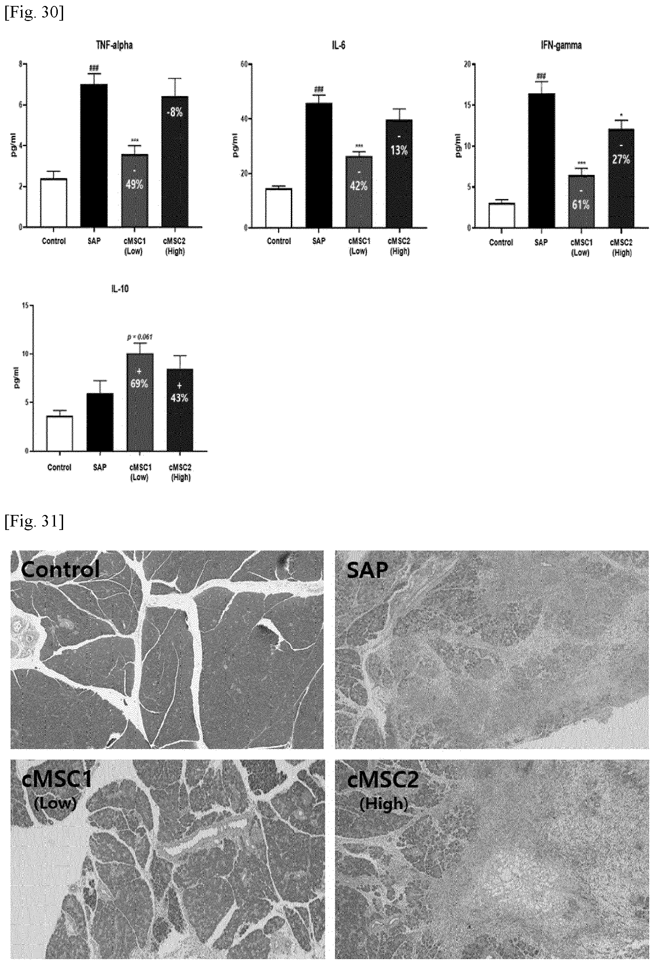

FIG. 30 is a view showing the results of identifying the changes in inflammatory cytokines TNF-α, IL-6, IFN-y and anti-inflammatory cytokine IL-10 according to cMSC1 and 2 treatment in an acute pancreatitis animal model (All values are presented as means with standard error of mean (SEM) P value = *, < 0.05; **, < 0.01; ***, < 0.001 vs. SAP group and ###, < 0.001 vs. control group).
FIG. 31 is a view showing the results of histopathological analysis according to cMSC1 and 2 treatment in an acute pancreatitis animal model.

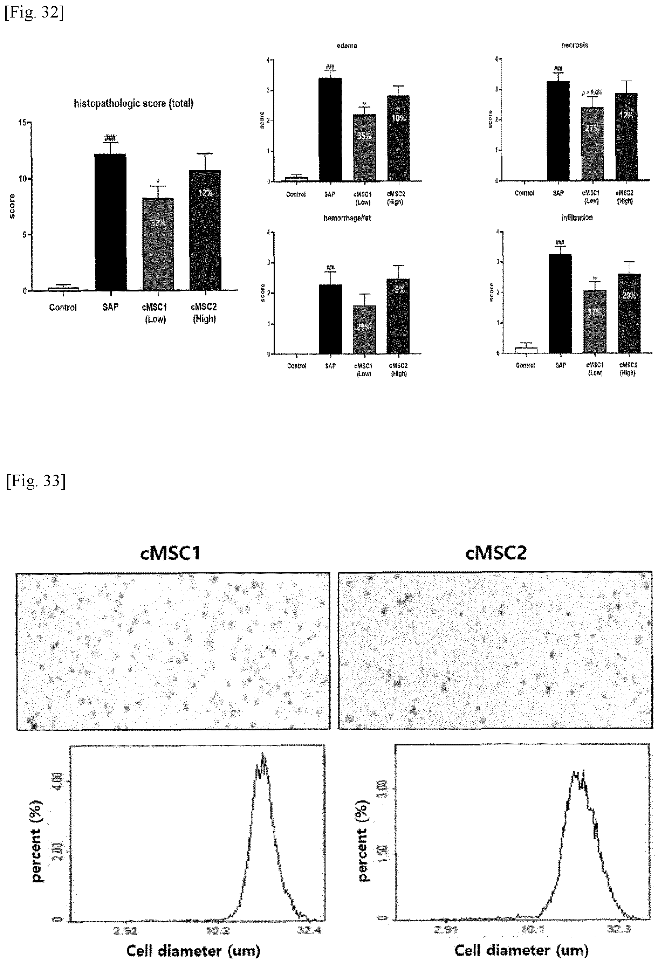

FIG. 32 is a view showing the results of identifying the histopathologic score, edema, necrosis, hemorrhage, and inflammatory infiltration scores according to cMSC1 and 2 treatment in an acute pancreatitis animal model (All values are presented as means with standard error of mean (SEM) P value = *, < 0.05; **, < 0.01; ***, < 0.001 vs. SAP group and ###, < 0.001 vs. control group).
FIG. 33 is a view showing the results of identifying the cell sizes of cMSC1 cultured by the improved method and cMSC2 cultured by the conventional method through a Nucleo Counter NC-250 device.

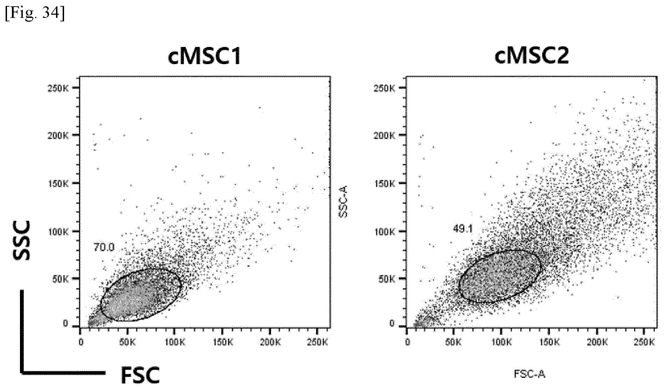

FIG. 34 is a view showing the results of identifying the cell size distribution of cMSC1 and cMSC2 using a flow cytometer.

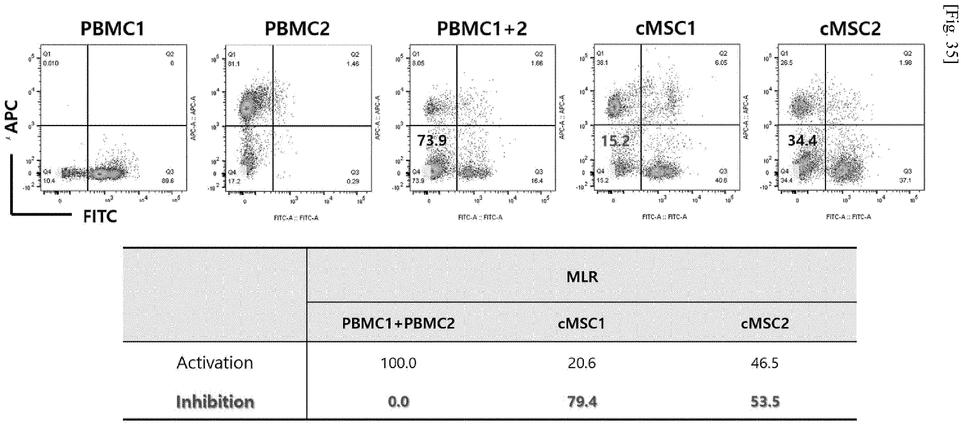

FIG. 35 is a view showing the results of identifying the immune cell inhibition ability of cMSC1 and cMSC2 by a mixed lymphocyte reaction (MLR) method (PBMC; peripheral blood mononuclear cell).

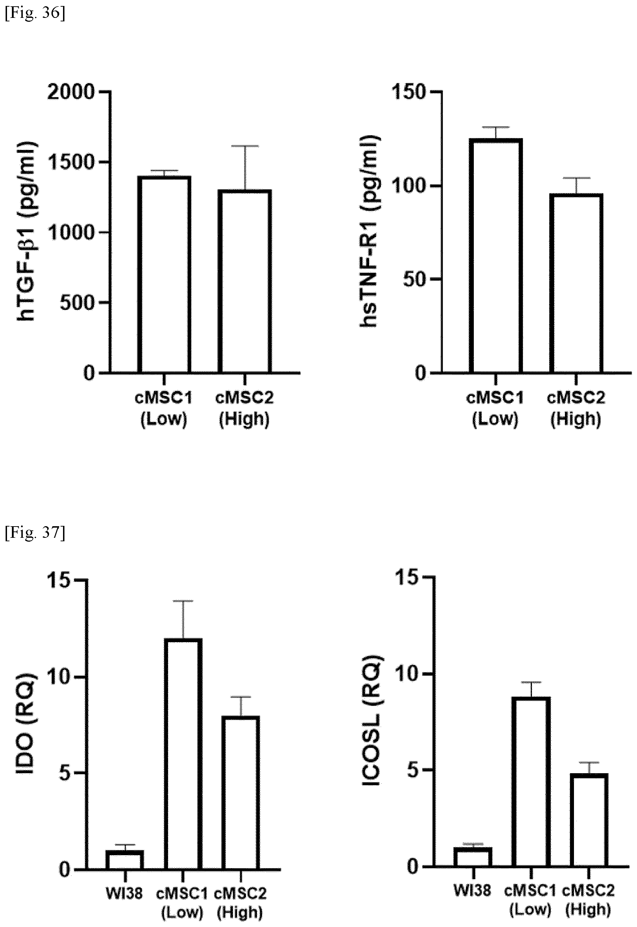

FIG. 36 is a view showing the results of identifying the secretion amount of hTGF-β1 and hsTNF-R1 (soluble tumor necrosis factor receptor 1) in the culture medium of cMSC1 and cMSC2.
FIG. 37 is a table showing the results of comparing the expression changes of the immune-related markers IDO (indoleamine 2,3-dioxygenase) and ICOSL (induced T cell co-stimulator ligand) expressed in cMSC1 and cMSC2 based on WI38 cells.

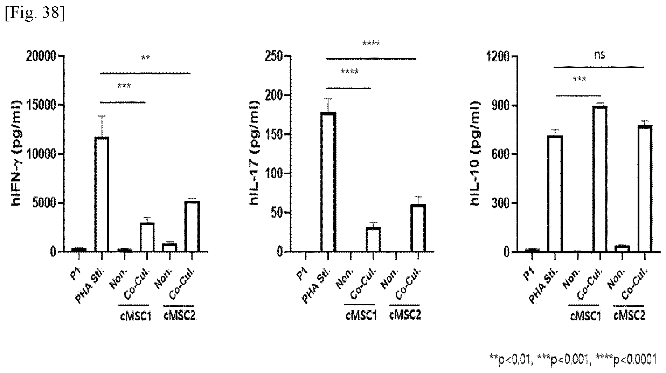

FIG. 38 is a view showing the results of identifying IFN-y, IL-17, and IL-10 secreted from a culture medium co-cultured with immune cells and cMSC1 and cMSC2.

### [Modes of the Invention]

The present disclosure is directed to a pharmaceutical composition for use in preventing or treating pancreatitis, the pharmaceutical composition comprising monoclonal stem cells (cMSC1) obtained through an improved method of subfractionation culture and proliferation of mesenchymal stem cells.

In addition, the present disclosure is directed to a method of producing a pharmaceutical composition comprising monoclonal stem cells obtained through an improved method of subfractionation culture and proliferation of mesenchymal stem cells.

In addition to the advantages of the subfractionation culturing method that can obtain stem cells quickly and without contamination, the monoclonal stem cells, which are active ingredients of the present disclosure, are stem cells obtained through an improved subfractionation culturing method capable of obtaining a large quantity of desired monoclonal stem cells in a short time without the need for a working cell bank (WCB) production process by rapid proliferation of monoclonal stem cells, preferably monoclonal mesenchymal stem cells. The monoclonal stem cells obtained through the above method are stem cells with enhanced therapeutic effects on pancreatitis compared to the stem cells obtained through the conventional subfractionation culturing method.

Hereinafter, the present disclosure will be described in detail.

An exemplary embodiment of the present disclosure provides a pharmaceutical composition for use in preventing or treating pancreatitis, the pharmaceutical composition comprising monoclonal stem cells obtained by steps of 1) culturing bone marrow isolated from a subject in a first vessel; 2) transferring only a supernatant from the first vessel to a new vessel and culturing the supernatant; 3) culturing cells present in the new vessel and obtaining a supernatant; 4) obtaining monoclonal stem cells by repeating steps 2) and 3) at least once using the supernatant of step 3) as the supernatant of the first vessel of step 2); 5) inoculating the monoclonal stem cells of step 4) in a medium at a cell density of 50 to 1,000 cells/cm² to culture the cells at passage 1; and 6) inoculating the subcultured monoclonal stem cells of step 5) in a medium at a cell density of 50 to 1000 cells/cm² to culture the cells at passages 2 to 8, wherein the culture medium in the step 5) and 6) further includes an antioxidant.

The culture in steps 2) and 3) is performed at 30 to 40°C for 4 hours or less, preferably for 1 to 3 hours, and more preferably for 1 hour 30 minutes to 2 hours 30 minutes. The repeated culture is performed at 30 to 40°C for 4 hours or less (preferably for 1 to 3 hours, more preferably for 1 hour 30 minutes to 2 hours 30 minutes) then is performed at 30 to 40°C for 12 to 36 hours (preferably for 18 to 30 hours) to be repeated 2 or 3 times. Subsequently, the culture is performed at 30 to 40°C for 24 to 72 hours and 36 to 60 hours (preferably for 36 to 60 hours). Each supernatant may be transferred to a new culture vessel to perform the next experiment.

According to one embodiment of the present disclosure, a brief summary of the isolation method is as follows.

Collection of bone marrow → Culture at 37°C for 2 hours → Culture at 37°C for 2 hours → Culture at 37°C for 2 hours → Culture at 37°C for 24 hours →
Transfer of only supernatant into new container
Transfer of only supernatant into new culture container coated with or without collagen (1)
Same as (1)
Same as (1)

Culture at 37°C for 24 hours → Culture at 37°C for 48 hours → Identification of growth on bottom of culture container → Culture at 37°C for 3 to 5 days → Identification of single cell group formation
Same as (1)
Same as (1)

The cultured cells form monoclonal cell groups. These monoclonal cell groups may be isolated and then subcultured. The present disclosure includes a subculture step of step 5) and step 6) in addition to the conventional subfractionation culturing method.

The term "subfractionation culturing method" used herein refers to a method of isolating stem cells according to specific gravity, indicating a process in that first, human bone marrow is extracted and cultured in a cell culture medium, then, only a supernatant is obtained, transferred to a culture vessel with or without treatment of a coating agent, and cultured, and then the same processes are repeated several times. Such a subfractionation culturing method is characterized by repeatedly obtaining and culturing the supernatant without centrifugation. It is advantageous that monoclonal stem cells, preferably monoclonal mesenchymal stem cells may be obtained without contamination of other cells finally.

Steps 1) to 4) of steps 1) to 6) of the present disclosure may be performed in the same or equivalent to the subfractionation culturing method described in Korean Patent Application No. 10-2006-0075676, US Patent Application No. 12/982,738, or Korean Patent Application No. 10-2013-7020033, and Korean Patent Application No. 10-2006-0075676.

In addition, conventionally, Korean Patent Application No. 10-2013-7020033 and US Patent Application No. 12/982,738 disclose a method of obtaining cells in connection with the treatment of pancreatitis, including: (i) obtaining a biological sample of bone marrow, peripheral blood, cord blood, fatty tissue sample, or cytokine-activated peripheral blood cells; (ii) allowing the biological sample of bone marrow, peripheral blood, cord blood, fatty tissue sample, or cytokine-activated peripheral blood cells to settle in a vessel; (iii) transferring supernatant which contains comparatively less dense cells compared to other cells from the vessel to another vessel in a serial manner at least two times; (iv) isolating less dense cells from the supernatant; and (v) administering the cells obtained in step (iv) to a subject suffering from pancreatitis, in which the bone marrow, peripheral blood, cord blood, fatty tissue sample, or cytokine-activated peripheral blood cells do not undergo centrifugation of greater than 1,000 rpm in steps (i) to (iii). The method is to obtain monoclonal stem cells only with a difference in density without centrifugation, and thus uses the conventional subfractionation culturing method of KR 10-2006-0075676.

However, the subfractionation culturing method of US Patent Application No. 12/982,738, Korean Patent Application No. 10-2006-0075676 and Korean Patent Application No. 10-2013-7020033 do not disclose a method of effectively obtaining monoclonal stem cells at a low passage, thereby obtaining monoclonal stem cells with remarkably improved therapeutic effects on pancreatitis.

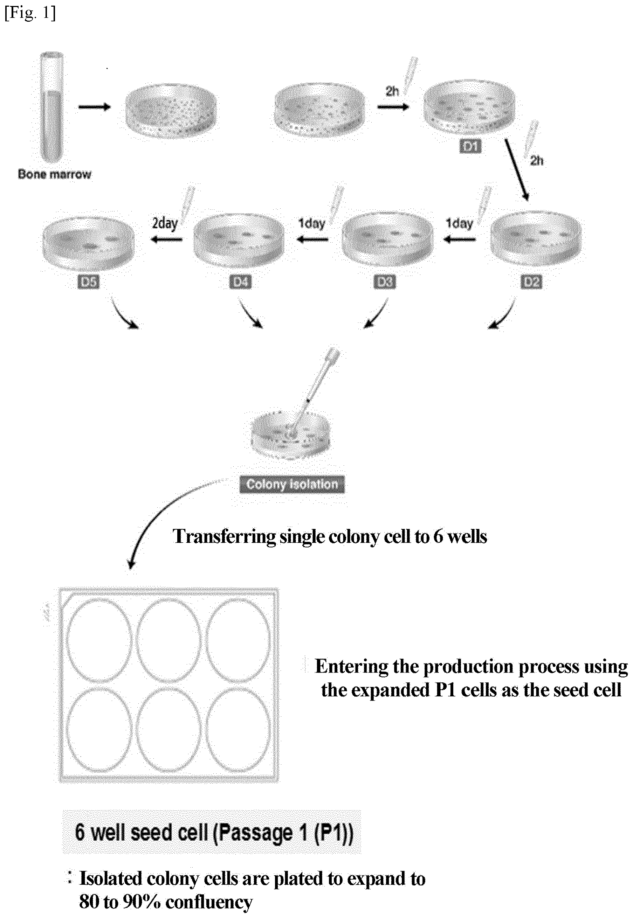

In the conventional subfractionation culturing method, as identified in FIG. 1, all cells obtained from a single colony were transferred to a 6-well plate and proliferated at 80-90% confluence, and then in order to obtain many cells without recognition of density control by using the proliferated passage 1 (P1) cells as seed cells, high-density culture was performed at 4,000 cells/cm².

The present disclosure relates to an "improved subfractionation culturing method" based on the fact that stem cells with excellent effects of preventing or treating pancreatitis may be effectively obtained by controlling the cell density in culture after passage 2, in which it is characterized in that the culturing after the seed cell is different from the conventional subfractionation culturing method. For example, specifically, the method includes the step of 5) inoculating the monoclonal stem cells of step 4) in a medium at a cell density of 50 to 1,000 cells/cm² to culture the cells at passage 1; and the step of 6) inoculating the subcultured monoclonal stem cells of step 5) in a medium at a cell density of 50 to 1000 cells/cm² to culture the cells at passages 2 to 8, wherein the culture medium in the step 5) and 6) further includes an antioxidant. The improved subfractionation culturing method may induce rapid proliferation of monoclonal stem cells compared to the conventional subfractionation culturing method, and thus the final product may be obtained quickly, and preferably only with a culture of less than passage 10 such as P2 to P8, a master cell bank (MCB) may be prepared, and monoclonal stem cells exhibiting excellent pancreatitis prevention or treatment effects may be obtained.

When the monoclonal stem cells are cultured at a high density of 4,000 cells/cm² as in the conventional process, the cell proliferation ability may be markedly decreased, the markers of the mesenchymal stem cells may be changed, and the differentiation potential of stem cells may be eliminated. Thus, the monoclonal stem cells obtained through the improved subfractionation culturing method may be cultured at a low density, that is, at the cell density of 50 to 1,000 cells/cm².

When monoclonal mesenchymal stem cells are cultured at a cell density of 1,000 cells/cm² or less, the cell proliferation ability is remarkably high over an extended period of culture compared with mesenchymal stem cells cultured at a high density of 4,000 cells/cm². Thus, there is an advantage in that a large amount of monoclonal cells which is desired may be rapidly obtained without repeating a large number of passages. Accordingly, the improved subfractionation culturing method of the present disclosure may perform the subculture step after seed cell at passage 10 or less, preferably passage 8 or less. Compared to a culture of up to passage 25 in order to secure a sufficient number of cells in the conventional subfractionation culturing method, it has an advantage of mass-producing monoclonal stem cells only with a small number of subcultures.

Further, when the monoclonal mesenchymal stem cells are cultured at the above-mentioned cell density, the cells have the advantage that their DNA may be less damaged, and they are less aged, thereby effectively maintaining the differentiation potential of the stem cells. Thus, monoclonal mesenchymal stem cells may be rapidly and quickly obtained to have excellent stem cell properties.

In addition, the monoclonal stem cells obtained according to the method of the present disclosure exhibit excellent pancreatitis prevention or treatment effects compared to the monoclonal stem cells cultured at a high density such as 4,000 cells/cm².

The medium used in the present disclosure may include a medium supplemented with an antioxidant, or a medium containing an antioxidant.

An antioxidant is added to the medium to perform the culture. α-MEM containing an antioxidant can be used to perform the culture.

The antioxidants may include, without limitation, antioxidants that can be used in cell cultures. They may include one or more selected from the group consisting of glutathione, cysteine, cysteamine, ubiquinol, beta-mercaptoethanol and ascorbic acid (AA). When the medium is supplemented with an antioxidant, the antioxidant may be added at a concentration of 10 to 50 µg/ml, preferably 10 to 30 µg/ml, and more preferably 25 µg/ml.

In one embodiment of the present disclosure, α-MEM is used as a medium containing ascorbic acid as an antioxidant.

According to the method of the present disclosure, since monoclonal stem cells may be proliferated very effectively, a process of producing WCB using MCB may be omitted. This is a simplification of the process compared to the conventional subfractionation culturing method that needs to involve a process of producing WCB after MCB production.

When a medium with an antioxidant is used as a culture medium of the present disclosure, the culture medium may be added with gentamicin as an antibiotic.

The mesenchymal stem cells obtained through the method of the present disclosure may finally be preferably P2 to P10 (excluding P10) mesenchymal stem cells, more preferably P2 to P8 mesenchymal stem cells, and even more preferably P2 to P6 mesenchymal stem cells. This indicates that these stem cells are obtained at a lower passage than the conventional process in which mesenchymal stem cells of at least P10 to P12 are obtained as a final product, and a large amount of mesenchymal stem cells rapidly proliferated at a low passage through cell inoculation density regulation is easily obtained.

In the present disclosure, the monoclonal stem cells (hereinafter referred to as "cMSC1") obtained by the improved subfractionation culturing method as described above with low density and antioxidant conditions, preferably 1,000 cells/cm² or less, are smaller in cell size and may be formed homogeneously compared to the monoclonal stem cells (hereinafter referred to as "cMSC2") obtained by the conventional subfractionation culturing method, and are capable of increasing the survival rate of mild type as well as severe type acute pancreatitis, alleviating the weight gain of the pancreas due to edema, and effectively reducing the increase in digestive enzymes and inflammation-related enzymes that are increased due to acute pancreatitis.

The term "pancreatitis" used herein refers to destruction of pancreatic secretory parenchyma and overall inflammation of the pancreas by pancreatic enzymes, including both chronic pancreatitis and acute pancreatitis, and may include acute pancreatitis and mild and severe acute pancreatitis without limitation.

The monoclonal stem cells obtained through the improved subfractionation culturing method of the present disclosure may also efficiently prevent or treat not only pancreatitis but also diseases resulting from pancreatitis, such as one or more diseases selected from the group consisting of lung injury, sepsis, renal failure, pleural effusion, multiple organ failure, and multiple organ damage.

The monoclonal stem cells obtained through the method of the present disclosure may improve at least one pancreatitis pathological condition selected from the group consisting of edema, necrosis, hemorrhage, and inflammatory infiltration.

cMSC1s, the monoclonal stem cells obtained through the improved subfractionation culturing method of the present disclosure, are remarkably excellent in terms of all activities of an improvement in survival rate of pancreatic cells, a decrease in alpha-amylase or lipase activity, a decrease in myeloperoxidase (MPO) activity, an improvement in neutrophil infiltration and inflammation, a decrease in inflammatory cytokine secretion, and an increase in anti-inflammatory cytokine secretion as compared to cMSC2 obtained by the conventional subfractionation culturing method, and are excellent in improving at least one pathological condition of pancreatitis selected from the group consisting of edema, necrosis, hemorrhage, and inflammatory infiltration. This difference in the therapeutic effect on pancreatitis is a remarkable effect of only the monoclonal stem cells of the present disclosure obtained by the improved subfractionation culturing method, and may result from enhanced at least one ability selected from the group consisting of TGF-β1 secretion ability, sTNF-R1 secretion ability, IDO expression ability, and ICOSL expression ability compared to cMSC2 obtained by the conventional method.

The pharmaceutical composition for use of the present disclosure may be prepared by including at least one pharmaceutically acceptable carrier in addition to the active ingredient for administration. The pharmaceutically acceptable carrier included in the pharmaceutical composition for use of the present disclosure is commonly used in preparation, and includes lactose, dextrose, sucrose, sorbitol, mannitol, starch, gum acacia, calcium phosphate, alginates, gelatin, calcium silicate, micro-crystalline cellulose, polyvinylpyrrolidone, cellulose, water, syrup, methyl cellulose, methyl hydroxybenzoate, propyl hydroxybenzoate, talc, magnesium stearate, and mineral oil, but is not limited thereto. In addition to the above ingredients, the pharmaceutical composition for use of the present disclosure may further include a lubricant, a wetting agent, a sweetener, a flavor enhancer, an emulsifying agent, a suspension agent, a preservative, and the like.

A dose of the pharmaceutical composition for use of the present disclosure may vary depending on the formulation method, administration manner, administration schedule and/or administration route of the pharmaceutical composition, and may vary according to various factors including the type and degree of the reaction to be achieved via administration of the pharmaceutical composition, the type, age, weight, general health conditions, the symptoms or severity of diseases, gender, diet, and excretion of subject to which the composition is administrated, drugs used concurrently in the subject or in combination for transplantation, and ingredients of other compositions, and similar factors well known in the medical field. The effective dose may be easily determined and prescribed for desired treatment by those of ordinary skill in the art.

The dose of the pharmaceutical composition for use of the present disclosure may be, for example, 1 mg/kg to 1,000 mg/kg per day, but the dose is not intended to limit the scope of the present disclosure in any way.

The administration route and administration manner of the pharmaceutical composition for use of the present disclosure may be independent of each other, the administration method is not particularly limited, and the administration route and the administration manner may be an arbitrary administration route and administration route as long as they enable the pharmaceutical composition to reach the desired corresponding site.

The pharmaceutical composition may be administered orally or parenterally. The parenteral administration may be, for example, intravenous administration, intraperitoneal administration, intramuscular administration, transdermal administration, or subcutaneous administration, and the pharmaceutical composition may be applied or sprayed on a disease site, or inhaled, but are not limited thereto.

The term "subject" used herein includes a subject in need of preventing or treating pancreatitis, and may be a mammal or a mammal other than a human.

In addition, an exemplary embodiment of the present disclosure provides a method of producing a pharmaceutical composition comprising monoclonal stem cells, the method comprising steps of 1) culturing bone marrow isolated from a subject in a first vessel; 2) transferring only a supernatant from the first vessel to a new vessel and culturing the supernatant; 3) culturing cells present in the new vessel and obtaining a supernatant; 4) obtaining monoclonal stem cells by repeating steps 2) and 3) at least once using the supernatant of step 3) as the supernatant of the first vessel of step 2); 5) inoculating the monoclonal stem cells of step 4) in a medium at a cell density of 50 to 1,000 cells/cm² to culture the cells at passage 1; and 6) inoculating the subcultured monoclonal stem cells of step 5) in a medium at a cell density of 50 to 1000 cells/cm² to culture the cells at passages 2 to 8, wherein the culture medium in the step 5) and 6) further includes an antioxidant.

According to the present disclosure, the monoclonal stem cells exhibiting excellent pancreatitis prevention or treatment effect compared to the monoclonal stem cells obtained by the conventional subfractionation culturing method may be obtained rapidly and easily without WCB production.

In the production method of the present disclosure, the culture of step 5) and step 6) above is performed by inoculating the monoclonal stem cells in a medium at a cell density of 1,000 cells/cm².

In addition, in the production method of the present disclosure, the medium in step 5) and step 6) is a medium supplemented with an antioxidant.

In the production method of the present disclosure, the contents described above may be equally applied, and overlapping contents are omitted in order to avoid the complexity of the description.

Hereinafter, the present disclosure is described in detail with the examples.

### <Example 1> Establishment of Improved Subfractionation Culturing Method

In order to produce the monoclonal mesenchymal stem cells exhibiting superior effects on pancreatitis, an improved method of subfractionation culture and proliferation of mesenchymal stem cells was used. The improved method of subfractionation culture and proliferation of mesenchymal stem cells varies the cell density and culture medium in the culture conditions of the subfractionation culturing method disclosed in Korean Patent Application No. 10-2006-0075676. In the experiments as below, the cell culture densities of the monoclonal mesenchymal stem cells (MSCs) obtained by the subfractionation culturing method were varied in 50 cells/cm² (low density), 1,000 cells/cm² (medium density) and 4,000 cells/cm² (high density), respectively, thereby analyzing the characteristics of the cells.

### 1.1. Identification of Morphological Changes of MSC According to Cell Density

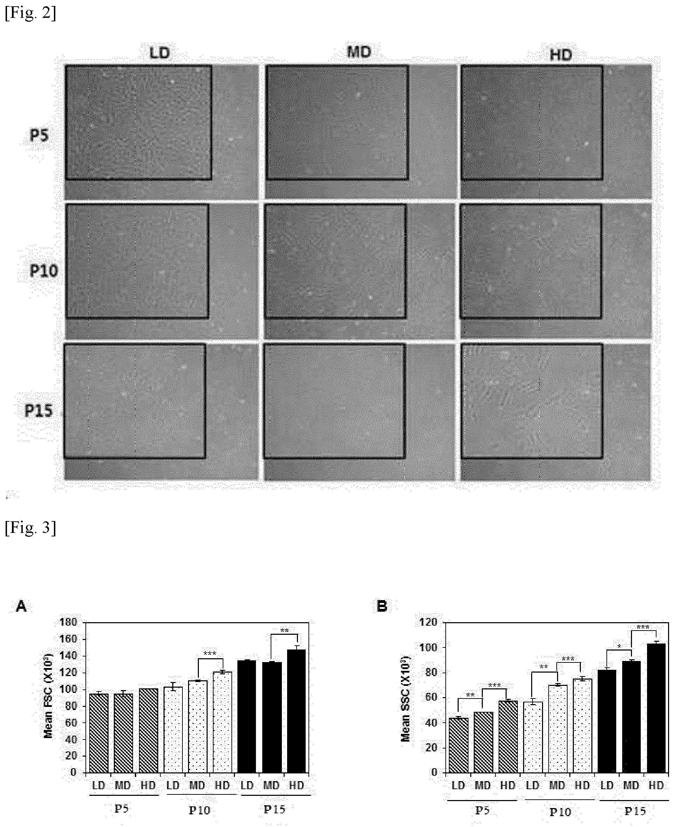

First, the experiments were conducted to identify the morphological changes of MSCs according to cell density in long-term culture. The MSCs having passage 5 (P5), passage 10 (P10) and passage 15 (P15) were used to change the culture conditions. The MSCs were inoculated in LG-DMEM under the condition of low density, medium density and high density, respectively. Thereafter, the morphological changes of the cells were observed through a microscope to determine whether or not the stem cells were aged. The results are illustrated in FIG. 2.

As illustrated in FIG. 2, the cell size and morphological pattern of P5 and P10 were different according to the cell density. In particular, P15 cultured under a high density culture condition had flat and enlarged MSCs. This morphology form indicates the typical aging of MSCs, which identifies that the cell density is controlled in the long-term culture, resulting in the control of MSC's aging.

### 1.2. Identification of MSC Size and Granularity According to Cell Density

In order to further identify the change of stem cells according to the cell density, the cell size and granularity, which were known to be increased in aged cells, were quantitatively analyzed by FACS analysis. Thus, the results are illustrated in FIG. 3.

As illustrated in FIG. 3, the cell size did not show a significant difference at P5, but P10 and P15 showed significant differences according to the cell density. In particular, the cell sizes in P10 and P15 were significantly increased under a culture condition of the high cell density, thereby further promoting aging of cells. Similarly, the cell granularity also was significantly increased as the cell density was increased in all passages. Therefore, it was identified that controlling cell density of MSC in long-term culture may be a factor to control aging of cells. Further, the cell culture density is lowered to improve the morphological changes in the late passage.

### 1.3. Identification of Aging of MSC According to Cultured Cell Density

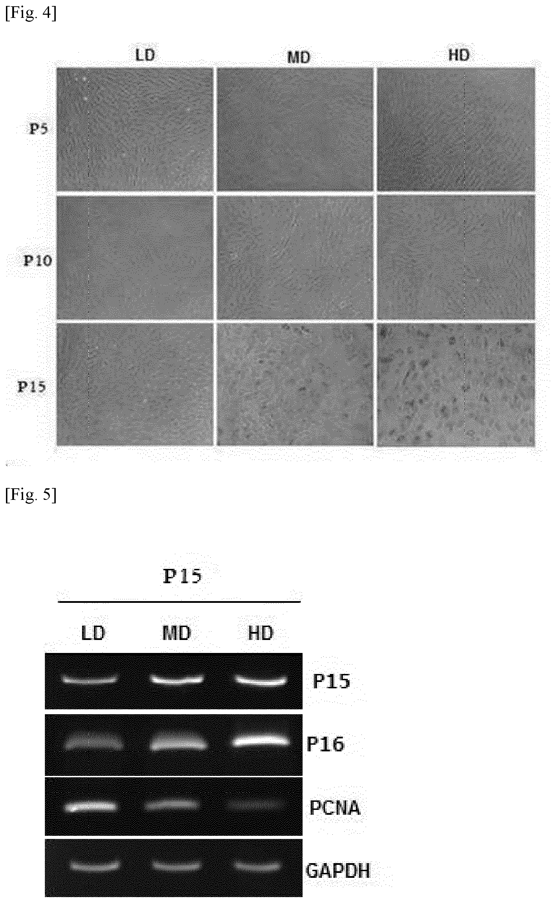

The beta-galactosidase-staining analysis was performed to identify whether the morphological changes confirmed in Examples 1.1 and 1.2 were actually an age-dependent phenomenon of MSC, which can selectively stain aging cells, and RT-PCR was performed to compare the expression of aging-related genes P15, P16 and PCNA gene, a proliferation marker. The results are illustrated in FIGS. 4 and 5, respectively.

As illustrated in FIG. 4, P5 and P10 did not have the staining of aged cells at all cell densities, but P15 had the staining of aged cells markedly increased as the cell density increased. As illustrated in FIG. 5, in P15, gene expression of CDK inhibitors P15 and P16, which are genes related to aging, was increased as the culture density of cells increased, and PCNA, which is a proliferation marker, decreased.

These results demonstrate that the morphological changes of MSCs are related to the aging of MSCs and that the control of the cell culture density may control the aging of MSCs.

### 1.4. Identification of Change of Proliferation Ability of MSCs According to Culture Cell Density

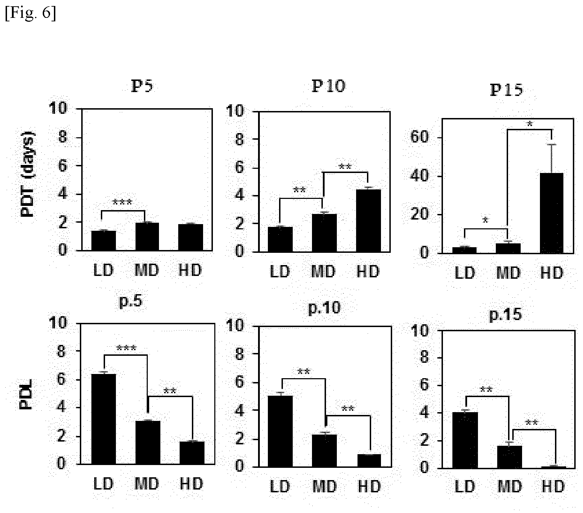

It is known that the proliferation ability of MSCs progressively decreases as cells are subcultured and aged. Therefore, the proliferation ability may be used as a criterion for identifying the aging of MSCs. Thus, the proliferation ability of MSCs according to the cell culture density was compared during long-term cell culture. The proliferation ability of each cell was determined by calculating the proliferation rate according to each passage using the number of cells which were initially inoculated and the number of cells which were obtained after the culture was completed. The results are shown in Table 1 and FIG. 6.

**[Table 1]**

| **Fold Increase** | | | |
|---|---|---|---|
| **(cell/cm²)** | **P5** | **P10** | **P15** |
| **50** | 88.4±6.5 | 34.3±5.0 | 16.4±1.3 |
| **1000** | 8.5±0.3 | 4.9±0.5 | 3.1±0.4 |
| **4000** | 3.0±0.1 | 1.9±0.1 | 1.1±0.1 |

As shown in Table 1, the fold increases were 88.4, 34.3 and 16.4 at P5, P10 and P15 in MSCs cultured at a low density. Meanwhile, the fold increases were 8.5, 4.9 and 3.1 in MSCs cultured at a medium density, and the fold increases were 3.0, 1.9, and 1.1 in MSCs cultured at a high density. As illustrated in FIG. 6, the PDT and the PDL also had the same pattern as the fold increase. These results indicate that the proliferation ability of MSCs may be maintained by lowering the cell density in long-term MSC culture and that even though performing the same subculture, the aging of MSCs may be inhibited and the lifespan of MSCs may be prolonged.

### 1.5. Identification of Change of Differentiation Potential of MSCs According to Culture Cell Density

The differentiation potentials according to P5 to P15 cultures were compared to identify whether culture cell density affects the ability of stem cells. The ability of stem cells to differentiate into adipocytes and osteocytes was identified. Qualitative and quantitative analyzes were performed at each passage and density. Specifically, NCS (Newborn Calf Serum) (Gibco), 10⁻⁷ mol dexamethasone (Sigma), 0.5 mM IBMX (Sigma), 10 µg/ml insulin (Sigma), and 100 µM indomethacin (Sigma) were added to a high glucose DMEM culture medium to prepare the adipocyte differentiation medium, and then the experiment was performed. After 7 days of differentiation, it was identified by Oil red O histochemical staining. After the Oil red O histochemical staining, it was eluted with isopropyl alcohol and measured at 500 nm and quantitatively analyzed.

The osteoclast differentiation medium was prepared and used by adding FBS (Gibco), 50 µg/ml ascorbic 2-phosphate (Sigma), 10⁻⁸ mol dexamethasone (Sigma) and 10 mM beta-glycerophosphate (Sigma) to α-MEM culture medium. After 21 days of differentiation, it was identified by Alizarin red S histochemical staining. After Alizarin red S histochemical staining, it was eluted with 10% acetic acid, measured at 405 nm and quantitatively analyzed. The adipocyte differentiation potential and the osteoclast differentiation potential were identified as described above. The results are illustrated in FIG. 7.

As illustrated in FIG. 7, the adipocyte differentiation potential decreased overall as the passage progressed, but the difference according to the density was not apparent. On the other hand, the osteoclast differentiation potential significantly decreased in the P15 culture group under the condition of high density. These results show that the osteoclast differentiation potential of MSCs may be maintained better when culturing at a low cell density.

### 1.6 Antigen Profile Analysis of MSCs According to Culture Cell Density

Experiments were performed to identify whether the cell culture density also affects the stem cell antigen expression. Flow cytometry was performed to identify the changes in positive and negative antigen expression according to each passage and culture density. The results are shown in Table 2.

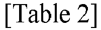

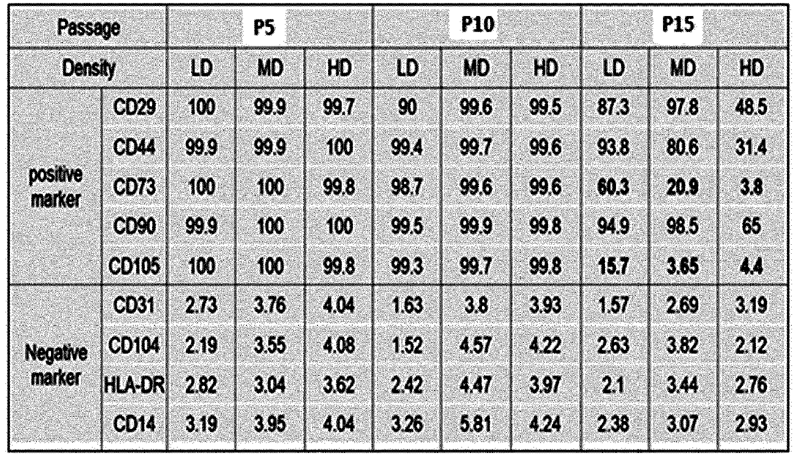

As shown in Table 2, the change of the expression of the negative marker was not clearly apparent, but the expression level of some positive markers was changed according to the cell culture density even in the same passage.

In particular, when cells were cultured at a high density in P15, the expression level of most positive markers was significantly decreased. Further, CD73 and CD105 showed negative expression. Thus, it was identified that cell culturing with a low cell density may be a critical factor.

### 1.7. Comparison of ROS Production and DNA Damage According to Culture Cell Density

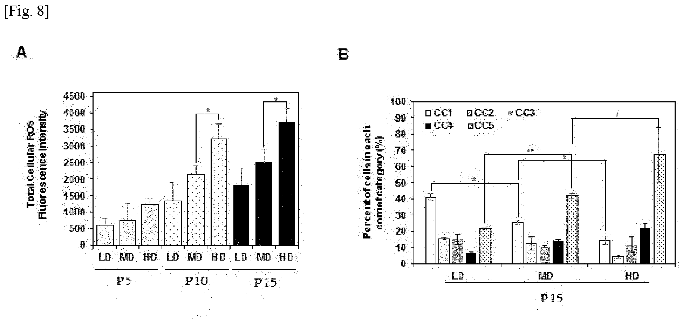

It is known that the decrease of mesenchymal stem cell function is associated with DNA damage. In particular, DNA damage induced by ROS, which is an active oxygen species, is known to promote aging of MSCs. Therefore, in order to identify whether total ROS production and DNA damage caused thereby are different according to culture density, fluorescence intensity analysis was performed to compare the total amount of cellular ROS according to passage and cell culture density. Comet analysis was performed to identify the degree of DNA damage. The results are illustrated in FIG. 8.

As illustrated in FIG. 8, total ROS production tended to increase as the cell culture density increased in all passages. In particular, ROS production significantly increased at P10 and P15 (A). In the comet analysis, the data were analyzed after classifying the data from CC1 with the weakest DNA damage to CC5 with the most severe DNA damage. The CC5 with the most severe DNA damage exhibited a significant increase as the cell culture density increased. On the other hand, the CC1 tended to decrease significantly as the cell density increased (B).

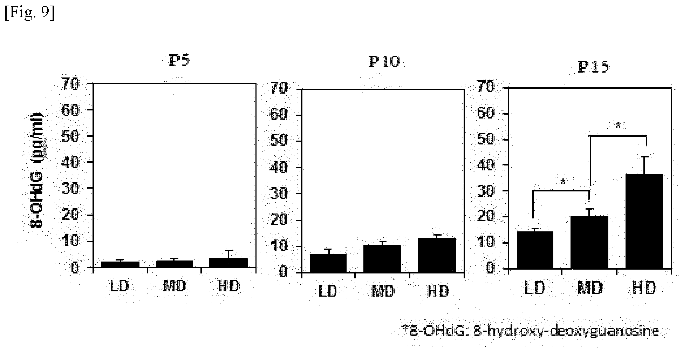

Further, in order to identify whether ROS caused DNA damage, an experiment was conducted to identify the concentration of 8-OHdG which may identify DNA damage caused by ROS. The analysis method of 8-OHdG is as follows. 50 µl of the DNA sample collected from each cell was placed on an 8-OHdG conjugate coated plate and incubated at room temperature for 10 minutes. Then, an anti-8-OHdG antibody was added thereto and the mixture was incubated at room temperature for 1 hour. After washing three times, secondary antibody-enzyme conjugate was added to each well, and the mixture was incubated at room temperature for another 1 hour. After washing three times, a substrate solution was added thereto, and the mixture was incubated at room temperature for 30 minutes. Finally, a stop solution was added thereto. The absorbance intensity thereof was measured at 450 nm. The results are illustrated in FIG. 9.

As illustrated in FIG. 9, as the cell culture density increased, the concentration of 8-OHdG was significantly increased in P15 group in which DNA damage was most severe. These results demonstrate that ROS produced under the culture condition of the high density caused DNA damage to increase, thereby promoting aging of MSCs.

These results show that lowering the cell culture density may play a role in protecting MSCs from DNA damage which is caused by increased ROS production ofMSCs.

### 1.8. Identification of MSC Proliferation and ROS Production Ability According to Antioxidant Treatment

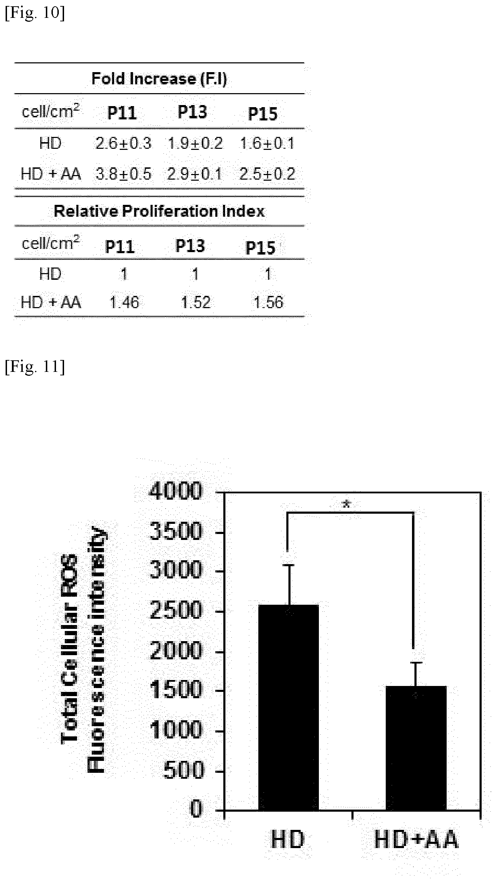

In order to identify whether ROS produced under the culture condition of high density affects the proliferation of MSCs, an experiment for eliminating ROS was performed. 25 µg/ml ascorbic acid, an antioxidant, was added to the medium under the culture condition of high density in P11 to P15. After the culture, the fold increase of proliferation rate was compared between the two groups. The results are illustrated in FIG. 10.

As illustrated in FIG. 10, the fold increase was 2.6, 1.9, and 1.6 at P11 to P15, respectively, in the high density culture condition. As passage number increased, the proliferation ability decreased, and as a result, aging began. However, treating with the antioxidant induced to maintain high proliferation ability at about 50% in all passages. In the antioxidant treatment group, the growth fold increase was 3.8, 2.9 and 2.5 in P11 to P15, respectively. The proliferation ability was maintained high even at P15.

At the endpoint P15, ROS levels were confirmed between the high density culture condition alone and high density culture condition + antioxidant treatment group. The results are illustrated in FIG. 11.

As illustrated in FIG. 11, the ROS level also decreased under the condition that proliferation was increased by treating ascorbic acid, an antioxidant. Therefore, MSC culture was preferably performed at a low cell density rather than a high density. ROS production induced by a high density of cell culture was eradicated with an antioxidant, thereby resulting in an increase of MSC proliferation ability. In other words, ROS at a high density inhibited MSC proliferation ability. As the cell density was lower, the ROS production was decreased, and the MSC proliferation ability was promoted.

To sum up, these results indicate that controlling the cell density at the density of 1,000 cells/cm² or less in culture conditions is important to maintain the proliferation, culture and stem cell ability of the monoclonal mesenchymal stem cells obtained through the subfractionation culturing method. Culture with an antioxidant inhibits oxidative stress induced by cell culture, thereby promoting MSC proliferation efficiently. In addition, when comparing culture under the same low cell density condition, stem cells of passage 10 or higher such as P15 showed significant changes in cell morphology when compared to stem cells of less than passage 10 such as passage 5, and results such as promoting aging of stem cells and decreasing differentiation potential were identified. It was identified that culture at a density of 1,000 cells/cm² or less with a low passage number of less than passage 10 was the most effective.

### <Example 2> Verification of Improved Subfractionation Culturing Method

Example 1 has identified that the control of cell density, passage control, and the addition of an antioxidant may be critical factors in the MSC culture obtained by the subfractionation culturing method. Therefore, the experiments were conducted to compare the proliferation ability of single colony MSCs and their effect of obtaining cells by varying the cell culture density of monoclonal mesenchymal stem cells obtained by the conventional method of the subfractionation culturing method described in Korean Patent Application No. 10-2006-0075676, and performing a subculture using a medium containing ascorbic acid as an antioxidant.

Example 1 of Korean Patent Application No. 10-2006-0075676, as previously disclosed, discloses a method of separating and culturing mesenchymal stem cells from the bone marrow through the subfractionation culturing method as shown in FIG. 1, and discloses that colonies, a single cell group, obtained through a subfractionation step were transferred to a culture vessel at 100 to 600 cells per well.

In addition, Korean Patent Application No. 10-2013-0106432 and US Patent Application No. 2012/0171168 disclose a method of separating and culturing bone marrow-derived mesenchymal stem cells using a subfractionation culturing method, and disclose that colonies are smeared at 50 to 100 cells/cm².

However, Korean Patent Application Nos. 10-2006-0075676, 10-2013-0106432 and US Patent Application No. 2012-0171168 merely disclose the configurations of counting single cell group colonies obtained by the subfractionation culturing method and transferring the same to a 6-well plate for culture, in other words, conditions for colony culture corresponding to passage 1, but do not disclose the configuration and effects of repetitive culture density control of individual cells other than colonies after passage 2. According to the conventional subfractionation culturing method described in the above application, culture of at least passage 10 needs to be performed to obtain a sufficient amount of monoclonal stem cells with effects of preventing, treating, or alleviating pancreatitis. On the other hand, in the improved subfractionation culturing method of the present disclosure, it is possible to effectively obtain a large amount of the monoclonal stem cells effective in treating the desired pancreatitis through low cell density conditions after passage 2 and a small number of subcultures of up to passage 8 or less.

Specifically, in this improvement method, colonies of passage 1 (P1) obtained through the subfractionation culturing method were cultured. Thereafter, in the subculture after passage 2 (P2), cells were seeded in 1,000 cells/cm² or less which is a low density. These were compared with the effect of 4,000 cells/cm² cells culture. Further, α-MEM medium containing antioxidant and LG-DMEM medium containing no antioxidant were used as a cell culture medium, thereby comparing their effects of cell proliferation.

Experimental groups for identifying the effect of the improved subfractionation culturing method are shown in Table 3 below. The process improvement parts of the improved subfractionation culturing method compared to the conventional subfractionation culturing method are schematically shown in FIG. 12.

As illustrated in FIG. 12, until the process of obtaining passage 1, the conventional subfractionation culturing method and the improved subfractionation culturing method proceeded in the same process. However, the improved subfractionation culturing method used the expanded passage 1 cell as a seed cell. Thus, the subculture process after the culture is different from the conventional subfractionation culturing method. The conventional subfractionation culturing method performs high-density subculture of 4,000 cells/cm² or more for the purpose of obtaining lots of cells without awareness of the density condition. However, the improved subfractionation culturing method is capable of obtaining the final products only with the culture after passage 2 and up to passage 8 or less by controlling the density of subculture to a low density of 1,000 cells/cm² or less. The culture process after passage 2 is shown in detail in FIG. 12.

**[Table 3]**

| **Group** | Culture density | Medium condition | **Passage** | Subculturing period **(day)** | Medium exchange |
|---|---|---|---|---|---|
| 400LG | 4000cells/cm² | LG-DMEM | **P2-P5** | 3-4 | x |
| 4000alpha | 4000cellls/cm² | alpha-MEM | | 3-4 | x |
| 1000LG | 1000cells/cm² | LG-DMEM | | 7 | O(every 3-4days) |
| 1000alpha | 1000cells/cm² | alpha-MEM | | 1 | O(every 3-4days) |

Cell lines of Table 3 above were separated by the subfractionation culturing method, which were named as SCM01 to SCM08, respectively.

### 2.1. Identification of Proliferation Effect According to Cell Line Density and Medium

The cells were cultured using the SCM01 to SCM08 cell lines. To identify the cell proliferation effect according to subculture of up to passage 5, which is less than passage 10, the cell number, population doubling time (PDT) and population doubling level (PDL) were compared, respectively. The results are illustrated in FIGS. 13 to 20.

As illustrated in FIGS. 13 to 20, the cell proliferation effect of all experimental groups inoculated and cultured with a cell density of 1,000 cells per cm² was superior to those of experimental groups inoculated and cultured with a cell density of 4,000 cells per cm². Furthermore, even in the same 1,000 cell density group, the 1,000-alpha experimental group cultured in α-MEM containing ascorbic acid as an antioxidant showed more significant cell proliferation effect than other groups.

### 2.2. Comparison of Proliferation Effect According to Cell Line Density

For a more accurate comparison of the proliferation rate according to the number of cultured cells, LG-DMEM and α-MEM, respectively, were set as a culture medium, and the cell proliferation effect according to the subculture of the cell inoculation density of 1,000 or 4,000 cells per cm² was compared. The results are illustrated in FIGS. 21 to 24.

As illustrated in FIG. 21, when the SCM01 to SCM08 cell lines inoculated with 1,000 cells/cm² were cultured in LG-DMEM, the proliferation rate of P2 to P5 was significantly higher than that of the group inoculated with 4,000 cells/cm². The proliferation rate of the group inoculated with 1,000 cells/cm² was at least 3.08 to at most 48.50 times compared with those of the group inoculated with 4,000 cells/cm² in passage 5 (P5).

Further, as illustrated in FIG. 22, the PDT value of 1,000 cells/cm² cell inoculation group was also lower or similar to those of 4,000 cells/cm² cell line inoculation in all cell lines, and the PDL value was higher than those of 4,000 cells/cm² cell inoculation in all cell lines.

Further, as illustrated in FIG. 23, when all SCM01 to SCM08 cell lines cultured in α-MEM were inoculated and cultured with 1,000 cells/cm², their tendency was similar to those of DMEM experimental groups. The proliferation rate of the group inoculated with 1,000 cells/cm² was at least 6.32 to at most 85.63 times compared with those of the group inoculated with 4,000 cells/cm² cell in passage 5 (P5). Further, as illustrated in FIG. 24, the PDT value of 1,000 cells/cm² cell inoculation group was also lower or similar to those of 4,000 cells/cm² inoculation in all cell lines, and the PDL value was higher than those of 4000 cells/cm² cell inoculation in all cell lines.

These results demonstrate that the inoculation with 1,000 cells/cm² or less may induce rapid proliferation of monoclonal mesenchymal stem cells compared to high density cell inoculation culture of 4,000 cells per cm².

### 2.3 Comparison of Proliferation Effect According to Culture Medium

Example 2.2 identified that 1,000 cells/cm² culture showed excellent proliferation effect compared with 4,000 cells/cm² culture. Therefore, the experiment was conducted to compare the cell proliferation effect while the number of cells was set as 1,000 cells/cm², and the medium varied as a variable. Thus, the proliferation effect was further verified according to the culture medium conditions. The results are illustrated in FIGS. 25 and 26.

As illustrated in FIG. 25, the cell proliferation rates were compared between α-MEM and DMEM. The results showed that the cell proliferation rate of the experimental group using α-MEM was at least 1.77 to 6.39 times higher compared with those of the experimental group using LG-DMEM. Further, as illustrated in FIG. 26, the PDT was low in all α-MEM experimental groups and the PDL was increased in all α-MEM experimental groups.

These results indicate that the cell proliferation efficiency may be maximized by culturing cells using a medium containing an antioxidant in addition to control of the cell inoculation density of 1,000 cells or less per cm² and culture of the cells in low passages of less than passage 10, such as passages 2 (P2) to 5 (P5).

### <Example 3> Establishment of Improvement Process

Examples, as described above, identified that the control of cell density and the addition of an antioxidant might be important factors in the MSC culture. In addition to the conventional process of the subfractionation culturing method described in Korean Patent Application Nos. 10-2006-0075676 and 10-2013-7020033, the improved process was established by varying the cell culture density and the medium conditions during subculture for effectively obtaining single colony mesenchymal stem cells at a low passage of less than passage 10. These are collectively shown in the following Table 4 (culture conditions using DMEM) and Table 5 (culture conditions using α-MEM).

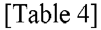

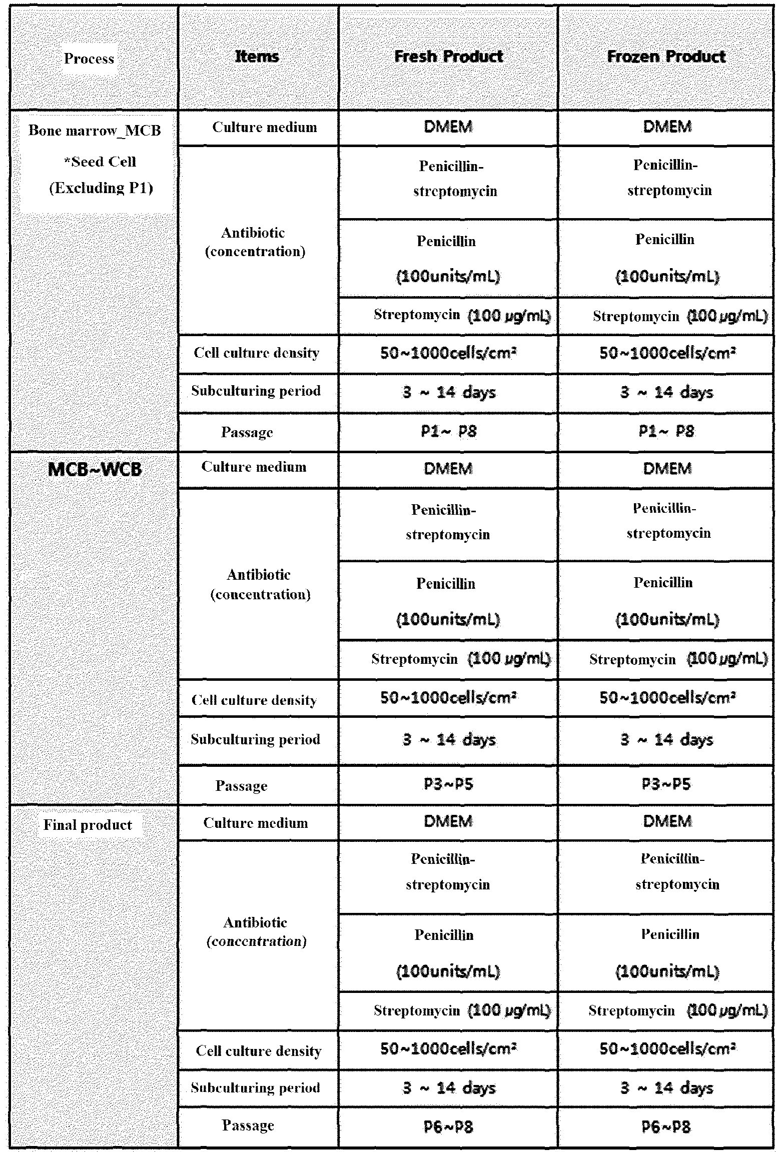

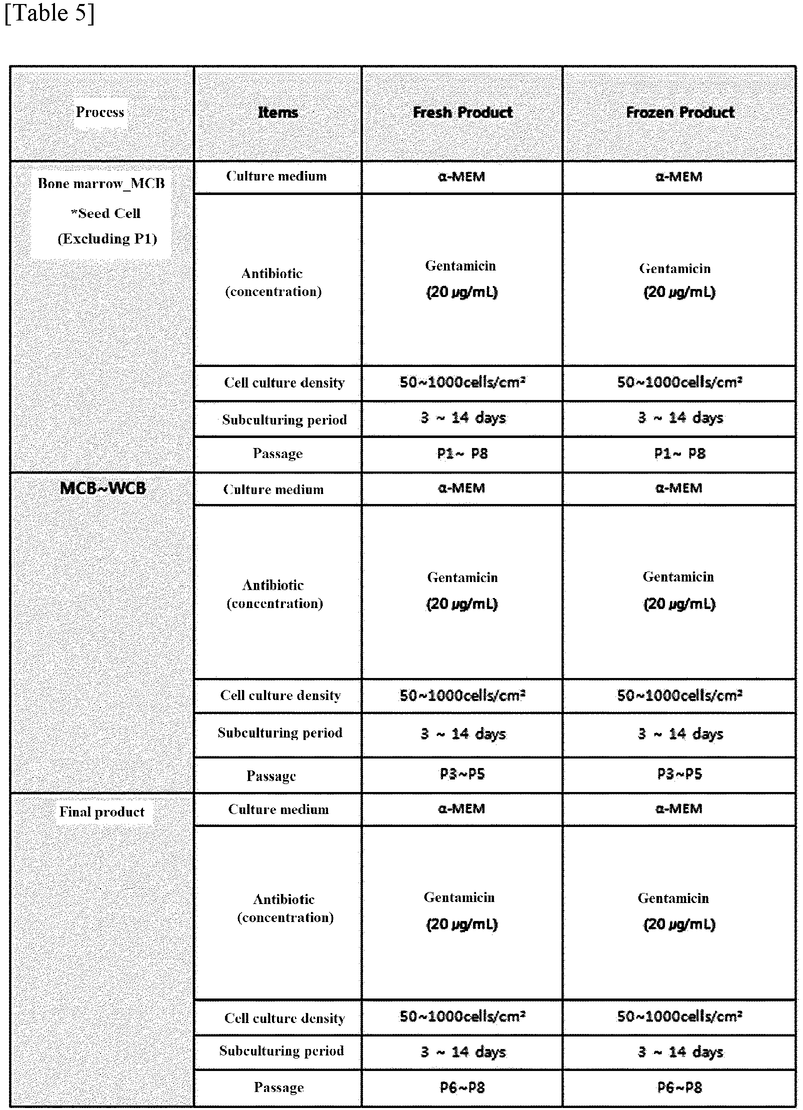

More specifically, the subfractionation culture process and proliferation culture of the bone marrow-derived mesenchymal stem cells of the present disclosure were performed as follows.

The hip of a bone marrow donor was anesthetized with local anesthetics. Then, the bone marrow was collected by piercing the needle into the hip bone. 14 ml of Dulbecco's modified Eagle's medium (DMEM, GIBCO-BRL, Life-technologies, MD, USA) containing 20% FBS and 1% penicillin/streptomycin, and 1 ml of human bone marrow were placed in a 100 mm culture vessel, and the mixture was cultured in a 5% CO₂ cell incubator at 37°C for 2 hours. After the culture, the culture vessel was slightly tilted to one side, and only the supernatant of the culture vessel was transferred to a new vessel while the cells attached to the bottom were prevented from falling down.

The same procedure was repeated one more time, and the resulting culture solution was transferred to a culture vessel (Becton Dickinson) coated with collagen on the bottom thereof and cultured at 37°C for 2 hours. The culture solution was transferred to a new vessel coated with collagen. After 24 hours, the culture solution was transferred to a new vessel. Again, after 24 hours, the culture solution was transferred to a new vessel. Finally, after 48 hours, the culture solution was transferred to a new vessel. Then, it was visually identified that remaining cells were grown and adhered to the bottom of the culture vessel. It can be assumed that cells which can come up to this step through the previous several subfractionation steps have a much smaller specific gravity of cells than other cells.

After about 10 days to about 14 days, the cells formed a single colony. These monoclonal cell groups were treated with trypsin to be isolated. Then, the cells were transferred to a 6-well culture vessel. The cells were cultured in a 5% CO₂ cell incubator at 37°C for 4 to 5 days. Then, when the cells were grown to about 80%, the cells were treated with 0.05% trypsin/1 mM EDTA (GIBCO-BRL), thereby obtaining the cells. Then, the cells were transferred to a T175 culture vessel and subcultured at a low cell density.

When the cells were cultured at a cell density which was lowered to 1,000 cells/cm² at passages 2 (P2) to 5 (P5) of less than passage 10, preferably passage 8 or less but all the other processes were regulated in the same manner, the proliferation ability and stem cell characteristics of MSCs were excellently maintained to induce efficient proliferation even in the same passage. In particular, when the cells were cultured at a lowered cell density, it may exclude a process of producing a WCB in MSCs, which is required in the conventional process, thereby shortening the cell production period efficiently. In particular, when the passage is reduced, cells with relatively less aging may be obtained in a large amount. It is expected that such cells are used as a therapeutic agent to lead to excellent therapeutic efficacy.

Further, when α-MEM supplemented with an antioxidant is used as a culture medium, the antioxidant treatment may effectively reduce the ROS stress induced in high density cell culture and restore the cell proliferation ability of MSCs, thereby shortening the cell passage significantly compared to the conventional process, and rapidly and stably obtaining single colony MSCs in a fresh state without aging, which maintains the characteristics of MSCs.

In summary, the low density cell culture may not only obtain a large number of cells in the short term, thereby simplifying the production process, but also obtain cells with non-aging to maintain the intact characteristics of MSCs in the long-term culture. Therefore, this leads to high quality stem cell production.

Accordingly, stem cells obtained through the improved method constructed through the above examples were used in the experiment for the treatment of pancreatitis below.

### <Example 4> Identification of Therapeutic Effects on Pancreatitis of Stem Cells obtained through Improved Subfractionation Culturing Metod

### 4.1 Preparation of cMSC1 and cMSC2

According to the improved subfractionation culturing method of Example 3, the cell culture density during subculture was set as 1,000 cells/cm² or less, and the monoclonal mesenchymal stem cells were obtained using α-MEM medium containing an antioxidant after being passaged three times. Gentamicin was added as an antibiotic, and the cell culture was performed using α-MEM culture medium *(see* Table 5). Hereinafter, the stem cells obtained through the improved subfractionation culturing method of low density of 1,000 cells/cm² or less and antioxidant conditions were named as "cMSC1." In addition, in order to compare the effect with the stem cells obtained by the improved subfractionation culturing method, the stem cells were obtained by the conventional subfractionation culturing method, which cultures cells by the culture method under the condition of the addition of no antioxidant and a density of 4,000 cells/cm² or more during subculture, and the 3 subcultured stem cells were named as "cMSC2."

### 4.2. Identification of Therapeutic Effects on Acute Pancreatitis according to cMSC1 Administration

### 4.2.1. Acute pancreatitis animal model construction and experimental method

In order to identify the therapeutic effect on acute pancreatitis according to cMSC1 administration, an acute pancreatitis animal model was set as shown in Table 6 below. The mice used in the experiment were purchased from SPF (Specific Pathogen Free) Rats (Koatech Co., Ltd.) and used, and 15 male rats aged 5 weeks for each group, a total of 60 rats, were used in the following experiments.

**[Table 6]**

| **Group** | **Experimental group** |
|---|---|
| **1** | **Normal control group (Control)** |
| **2** | **Vehicle (SAP)** |
| **3** | **cMSC1** (Induction of acute pancreatitis + cMSC1 administration group) |
| **4** | **cMSC2** (Induction of acute pancreatitis + cMSC2 administration group) |

To construct an animal model of acute pancreatitis, the abdomen of the animal was depilated using a clipper before surgery. Anesthesia was performed using Zoletil 50 (VIRBAC, France) and xylazine (Rompun^{®} Bayer AG, Germany), and additional anesthesia was performed when needed. After disinfecting the area to be incised widely using povidone and 70% alcohol, a median abdominal incision was performed. After exposing the duodenum, acute pancreatitis was induced by administering 3% sodium taurocholate, a substance that induces acute pancreatitis, into the pancreatic duct at a dose of 1 mL/kg.

After 4 hours from induction of pancreatitis in an animal model, 2 × 10⁶ cells of cMSC1 and cMSC2 were administered once 200 ul through the tail vein, and after 72 hours, blood and organs were excised and analyzed. FIG. 27 shows a schematic view of the construction of an acute pancreatitis animal model according to the present disclosure.

### 4.2.2. Identification of Pancreatic Cell Survival Rate according to cMSC1 and cMSC2 Treatment

In the animal model of acute pancreatitis prepared in Example 4.2.1, the injected cell survival rate was identified. cMSC1 and cMSC2 were administered through the tail vein at the same dose with the cell stabilizers shown in Table 7 below.

**[Table 7]**

| **Test substance name** | Dose **(cells/head)** | Cell survival rate | Administration route | Dosage **(mL/head)** |
|---|---|---|---|---|
| **cMSC1** | 2×10⁶ | 91% | Tail vein administration | 200ul |
| **cMSC2** | 2×10⁶ | 87.796 | | |

| Test substance name | Reagent name | Composition | Administration route | Dosage **(mL/head)** |
|---|---|---|---|---|
| Vehicle | Plasma Solution A | 67.5% | Tail vein administration | 200ul |
| | Human Serum Albumin | 22.5% | | |
| | DMSO (Clinical-grade) | 10% | | |

As identified in Table 7 above, as a result of measuring the survival rate upon injection of the test substances cMSC1 and cMSC2, it was identified that the survival rates were 91% and 87.7%, respectively, all of which were 85% or higher. In particular, the cMSC1 administration group showed a survival rate of 90% or more, thus identifying that the protective effect of pancreatic cells was more excellent.

### 4.2.3. Blood Biochemical Effects according to cMSC1 Treatment

In order to identify the blood biochemical effect according to cMSC treatment in the rats of Example 4.2.1 in which acute pancreatitis was induced, the pancreatitis marker level was checked. Specifically, two enzymes alpha-amylase and lipase were measured using a blood biochemical analyzer (7180 Hitachi, Japan) 72 hours after administration of cMSC1 or cMSC2, which is the end point of the test. The results are shown in FIG. 28.

As shown in FIG. 28, it was identified that the levels of alpha-amylase and lipase in the acute pancreatitis (SAP) model at 72 hours after administration of cMSC1 and 2 were significantly higher than those of the control group. The cMSC2 administration group showed no effect at the level of alpha-amylase compared to the SAP model, but it was identified that the increase in lipase was inhibited by about 13%. On the other hand, in the cMSC1 administration group obtained through the improved subfractionation culturing method, it was identified that the amylase level decreased by 51% compared to the SAP control group, and lipase also showed a significant level reduction of 64%.

In other words, it was identified that when cMSC1 was injected, both alpha-amylase and lipase, which are pancreatitis markers, were significantly reduced by about 51 to 64%. These results indicate that cMSC1 would very significantly reduce the alpha-amylase and lipase activities in serum.

### 4.2.4. Identification of Myeloperoxidase (MPO) according to cMSC1 treatment

Myeloperoxidase (MPO) is an enzyme that is abundantly expressed in neutrophil granulocytes, and is used as a predictor of myocardial infarction or acute myeloid leukemia and is a marker indicating the degree of inflammation. In order to identify whether the degree of infiltration and inflammation of neutrophils in the pancreatic tissue is improved according to the cMSC1 treatment of the present disclosure, myeloperoxidase (150 kDa) was measured according to the manufacturer's method using Myeloperoxidase activity assay kit (STA-803, Cell biolabs). The results are shown in FIG. 29.

As shown in FIG. 29, myeloperoxidase activity was increased by about 12 times compared to the control group in the group inducing acute pancreatitis, but significantly decreased by 65% or more in the cMSC1 treated group obtained by the improved subfractionation culturing method. These results show that cMSC1 exhibits a statistically significant and remarkable neutrophil reduction effect compared to the 8% reduction effect of the cell line group cMSC2 in the conventional process.

### 4.2.5. Analysis of inflammatory cytokines and anti-inflammatory cytokines according to cMSC1 treatment

In order to identify whether changes in inflammatory factors are induced by cMSC treatment in the rats of Example 4.2.1, which is an acute pancreatitis animal model, the expression change of inflammatory cytokines TNF-α, IL-6, IFN-y, and IL-10 was measured according to the manufacturer's method using the analysis tool of Table 8 below. The results are shown in FIG. 30.

**[Table 8]**

| Item | **Cat No.** | Manufacturer | Usage |
|---|---|---|---|
| **Rat TNF-α ELISA Kit** | SRTA00 | R&D Systems | Serum analysis |
| **Rat IL-6 ELISA Kit** | SR60008 | R&D Systems | |
| **Rat IFN-γELISA Kit** | SRIF00 | R&D Systems | |
| **Rat IL-10 ELISA Kit** | SR1000 | R&D Systems | |

As shown in FIG. 30, it was identified that in the acute pancreatitis animal model (SAP), the levels of TNF-α, IL-6, and IFN-y were significantly increased compared to the control group, but in the cMSC1 and cMSC2 treatment groups, the expression level was significantly decreased compared to the control group. In particular, the cMSC1 treatment group showed reduction effects of 49%, 42%, and 61%, respectively, in TNF-α, IL-6, and IFN-γ compared to the control group, thus identifying a remarkably excellent inhibitory effect on inflammatory cytokines. In addition, the anti-inflammatory cytokine IL-10 was increased in both the cMSC1 and cMSC2 treatment groups compared to the control group. In particular, the IL-10 level of cMCS1 increased by 69% compared to the control group, and thus it was identified that an increase in the expression of anti-inflammatory cytokines could be induced by cMSC treatment.

### 4.2.5. Histopathological Analysis according to cMSC1 treatment

The organs of the animal model of acute pancreatitis of Example 4.2.1 were excised, and histopathological analysis was performed to identify the change of the edema lesion according to the cMSC1 treatment. Specifically, blood was collected 72 hours after administration of the test substance, and then the animals were euthanized, and then the pancreas was excised and fixed with 10% neutral buffered formalin. Using the fixed tissue, general tissue processing procedures such as trimming, dehydration, paraffin embedding, and cutting were performed, and specimens for histopathological examination were prepared. Hematoxylin & Eosin (H&E) staining was performed, and histopathological changes were observed using an optical microscope (Olympus BX53, Japan). Histopathological evaluation was scored using Schmidt's score (A better model of acute pancreatitis for evaluating therapy, Schmidt J et al, 1992). The results are shown in FIGS. 31 and 32.

As shown in FIG. 31, it was identified that inflammation was found in SAP compared to the control group under the microscope, and the degree of inflammation was most improved in the cMSC1 treatment group.

As shown in FIG. 32, in the animal model of acute pancreatitis, histopathology scores, edema, necrosis, hemorrhage, and inflammatory infiltration scores all increased sharply compared to the control group, and all these sharp increases were alleviated by cMSC1 and cMSC2 treatment. In particular, it was identified that the cMSC1 treatment group exhibited a reduction effect of about 27 to 35% in all evaluation indicators compared to the control group, showing about twice the effect of the cMSC2 treatment group.

Overall, in the case of cMSC1 obtained by the improved subfractionation culturing method, it was identified that cMSC1 may increase pancreatic cell survival rate in acute pancreatitis and effectively improve both blood biochemical and histopathological lesions, thus exhibiting excellent effects in the treatment of acute pancreatitis. In addition, it was identified that the effect of cMSC1 on the treatment of acute pancreatitis was remarkably superior even when compared with cMSC2 obtained by the conventional subfractionation culturing method and subcultured at high density.

### <Example 5> Comparison of Stem Cell Characteristics obtained by Improved Subfractionation Culturing Method

### 5.1. Comparison of cMSC1 and cMSC2 Cell Characteristics

Since it was identified in Example 4 that the stem cells cMSC1 obtained by the improved subfractionation culturing method exhibited remarkably superior acute pancreatitis treatment effect compared to cMSC2 obtained by the conventional subfractionation culturing method, an experiment was conducted to compare the characteristics of these cells.

In the same manner as in Example 4.1, cMSC1 and cMSC2 were obtained, and these cells were cultured and the cell size was checked.

The cells and methods used in the experiment are shown in Table 9 below.

**[Table 9]**

| Cell name | Media used | Number of cells cultured per area | Culture period | Particulars |
|---|---|---|---|---|
| cMSC1 | α-MEM | 1000cells/cm² | 7 days | Addition of antioxidant to medium 1000a |
| cMSC2 | DMEM | 4000cells/cm² | 3-4 days | 4000LG |

In order to verify the difference in cell size between cMSC1 and cMSC2, the cell size was identified using a Nucleo Counter NC-250 device. The results are shown in FIG. 33 and Table 10.

**[Table 10]**

| **Cell Name** | **cMSC1** | **cMSC2** |
|---|---|---|
| **Estmated Cell diameter (um)** | **18.0** | **18.9** |
| **Cell Diameter Standard Deviation (um)** | **10.5** | **12.6** |

As shown in Table 10 and FIG. 33, it was identified that the two cells were different in size, and as a result of identifying the standard deviation of the cell diameter through the device, it was identified that the deviation of cMSC1 obtained by the improved subfractionation culturing method was smaller than that of cMSC2.

In addition, when the forward-scattered/side-scattered light (FSC/SSC) values were identically designated using a flow cytometer (FACS), it was identified whether the size of the cells was different also in flow cytometry. The results are shown in FIG. 34.

As shown in FIG. 34, it was identified that cMSC2 had a large cell size and thus cells were generally widely distributed, whereas cMSC1 had a small cell size and thus was distributed within 50 K of side-scattered light (SSC).

As described above, as the cell size of cMSC1 was smaller and homogeneously formed, the amount of cytokines secreted while mesenchymal stem cells are cultured was changed. Thus, it was identified that the therapeutic effect on acute pancreatitis of cMSC1 was further enhanced.

### 5.2. Identification of in vitro effect of cMSC1 and cMSC2

After culturing cMSC1 and cMSC2, which exhibit different effects on acute pancreatitis, *in vitro* experiments were performed to compare cell-specific activities. First, the inhibition rate of activated T cells was identified in the condition of mixed lymphocyte reaction (MLR). The experimental method is as follows. Two different donors' PBMCs were mixed to induce antigen-induced T cell activity (allogeneic MLR), and then cMSC1 or cMSC2 was added to identify whether T cell activity was inhibited. Human PBMC stained with each dye (CFSE and eFluor670) and cMSC1 or cMSC2 were co-cultured at a ratio of 4 : 1, respectively, and cultured for 8 days. The analysis was performed by flow cytometry using a FACS verse (BD Biosciences) device. The results are shown in FIG. 35.

As shown in FIG. 35, as a result of identifying the inhibition rate of activated T cells under mixed lymphocyte reaction conditions, both cells showed an inhibition rate of 50% or more under the condition of T cell : cMSC = 1 : 4. However, the cell line of an improved process (cMSC1) showed an inhibition rate of 79%, and the cell line (MSC2) of the previous process showed an inhibition rate of 53%, thus identifying a difference of about 26%.

cMSC1 and cMSC2 were cultured *in vitro,* and the expression levels of TGF-β1, sTNF-R1 (soluble tumor necrosis factor receptor 1), and immune-related markers IDO (indoleamine 2,3-dioxygenase), ICOSL (induced T cell co-stimulator ligand) in the culture medium of each cell line were compared. The results are shown in FIGS. 36 and 37.

As shown in FIG. 36, as a result of identifying the secretion amount of TGF-β1 and sTNF-R1 in the culture medium in which the two cell lines were cultured, TGF-β1 did not show a significant difference between the two cell lines, but it was identified that sTNF-R1 was secreted as high as 28 pg/ml in cMSC1, a cell line of an improved process.

In addition, as shown in FIG. 37, as a result of comparing the expression levels of IDO and ICOSL based on WI38 (human fibroblast) in a state where no stimulation was given, it was identified that the expression levels of two genes were about twice higher in cMSC1, the cell line of the improved process.

In addition, after co-culture under PHA (phytohemagglutinin) stimulation conditions, changes in inflammatory cytokines (IFN-y, IL-17) and anti-inflammatory cytokines (IL-10) were identified as culture media. In order to identify the amount of secretion of IFN-y, IL-17, and IL-10, PBMC (peripheral blood mononuclear cell) at a concentration of 1 × 10⁶ cells/well was smeared on a 24-well plate and an inflammatory reaction was induced with 1 ug/ml PHA. Specifically, the concentrations of IFN-y, IL-17, and IL-10 were measured in each experimental group by varying the presence or absence of the PHA treatment and the cMSC treatment. The results are shown in FIG. 38.

As shown in FIG. 38, as a result of co-culture with the cell line cMSC1 of the improved process, it was identified that IFN-y was inhibited by 74.3% and IL-17 was inhibited by 82.2% compared to the PHA stimulation condition, and in the cell line cMSC2 obtained by the conventional process, it was identified that IFN-y was inhibited by 55.4% and IL-17 was inhibited by 65.8%. In other words, it was identified that the inhibition rate of inflammatory cytokine was about 20% different between the two cell lines. In addition, as a result of identifying the amount of secretion of the anti-inflammatory cytokine IL-10 under the same conditions, IL-10 was increased by about 25% in the cell line cMSC1 of the improved process, whereas IL-10 was increased by 7% in the cell line cMSC2 of the previous process, thus identifying that cMSC1 further increased the secretion of anti-inflammatory cytokines by about three times more than that of cMSC2.

To sum up the above results, it was identified that the use of cMSC1 obtained through the improved process may effectively reduce the increase in digestive enzymes and inflammation-related enzymes that are increased due to acute pancreatitis. In addition, it was identified that the secretion of inflammatory cytokines was reduced and the secretion of anti-inflammatory cytokines was more remarkably increased, and significant results were also identified through histological analysis of the pancreas. In particular, cMSC1 according to the present disclosure is capable of maintaining excellent proliferation ability and stem cell characteristics to effectively maintain proliferation. Moreover, cMSC1 was identified to be more excellent in immunomodulatory ability, immune-related gene expression, , and secretion of inflammation and anti-inflammatory cytokines as compared to the cell line cMSC2 obtained by the previous process. Therefore, it can be understood that the cells obtained by the improved process may prevent and treat pancreatitis better than the cells obtained by the previous process.

## Claims

1. A pharmaceutical composition for use in preventing or treating pancreatitis, the pharmaceutical composition comprising monoclonal stem cells obtained by steps of:
1) culturing bone marrow isolated from a subject in a first vessel;
2) transferring only a supernatant from the first vessel to a new vessel and culturing the supernatant;
3) culturing cells present in the new vessel and obtaining a supernatant;
4) obtaining the monoclonal stem cells by repeating steps 2) and 3) at least once using the supernatant of step 3) as the supernatant of the first vessel of step 2) ;
5) inoculating the monoclonal stem cells of step 4) in a medium at a cell density of 50 to 1000 cells/cm² to culture the cells at passage 1; and
6) inoculating the subcultured monoclonal stem cells of step 5) in a medium at a cell density of 50 to 1000 cells/cm² to culture the cells at passages 2 to 8,
wherein the culture medium in the step 5) and 6) further includes an antioxidant.

2. The composition for use according to claim 1, wherein the subculture of step 5) or 6) is performed by inoculating the monoclonal stem cells in a medium at a cell density of 1,000 cells/cm².

3. The composition for use according to claim 1, wherein the pancreatitis is a chronic pancreatitis or an acute pancreatitis.

4. The composition for use according to claim 1, wherein the monoclonal stem cells improve at least one pancreatitis-related pathological condition selected from the group consisting of edema, necrosis, hemorrhage, and inflammatory infiltration.

## Patentansprüche

1. Pharmazeutische Zusammensetzung zur Verwendung bei der Vorbeugung oder Behandlung von Pankreatitis, wobei die pharmazeutische Zusammensetzung monoklonale Stammzellen umfasst, die mit folgenden Schritten erhalten werden:
1) Kultivieren von einem Individuum entnommenem Knochenmark in einem ersten Gefäß;
2) Überführen nur eines Überstands aus dem ersten Gefäß in ein neues Gefäß und Kultivieren des Überstands;
3) Kultivieren von im neuen Gefäß vorhandenen Zellen und Gewinnen eines Überstands;
4) Gewinnen der monoklonalen Stammzellen durch wenigstens einmaliges Wiederholen der Schritte 2) and 3) unter Verwendung des Überstands aus Schritt 3) als Überstand aus dem ersten Gefäß in Schritt 2);
5) Animpfen eines Mediums mit den monoklonalen Stammzellen aus Schritt 4) bei einer Zelldichte von 50 bis 1000 Zellen/cm² zum Kultivieren der Zellen bei Passagierung l; und
6) Animpfen eines Mediums mit den subkultivierten monoklonalen Stammzellen aus Schritt 5) bei einer Zelldichte von 50 bis 1000 Zellen/cm² zum Kultivieren der Zellen bei Passagierung 2 bis 8,
wobei das Kulturmedium in Schritt 5) und 6) ferner ein Antioxidationsmittel enthält.

2. Zusammensetzung zur Verwendung nach Anspruch 1, wobei die Subkultur in Schritt 5) oder Schritt 6) durchgeführt wird, indem ein Medium mit den monoklonalen Stammzellen bei einer Zelldichte von 1.000 Zellen/cm² angeimpft wird.

3. Zusammensetzung zur Verwendung nach Anspruch 1, wobei es sich bei der Pankreatitis um eine chronische Pankreatitis oder eine akute Pankreatitis handelt.

4. Zusammensetzung zur Verwendung nach Anspruch 1, wobei die monoklonalen Stammzellen zur Verbesserung wenigstens eines mit Pankreatitis verbundenen, aus der Gruppe bestehend aus Ödem, Nekrose, Blutung und entzündlicher Infiltration ausgewählten Krankheitszustands führen.

## Revendications

1. Composition pharmaceutique pour une utilisation dans la prévention ou le traitement d'une pancréatite, la composition pharmaceutique comprenant des cellule souches monoclonales obtenues par les étapes de :
1) culture de moelle osseuse isolée d'un sujet dans un premier récipient ;
2) transfert seulement d'un surnageant du premier récipient à un nouveau récipient et la culture du surnageant ;
3) culture de cellules présentes dans le nouveau récipient et l'obtention d'un surnageant ;
4) obtention des cellules souches monoclonales par répétition des étapes 2) et 3) au moins une fois en utilisant le surnageant de l'étape 3) comme le surnageant du premier récipient de l'étape 2) ;
5) inoculation des cellules souches monoclonales de l'étape 4) dans un milieu à une densité de cellules de 50 à 1 000 cellules/cm² pour cultiver les cellules au passage 1 ; et
6) inoculation des cellules souches monoclonales sous-cultivées de l'étape 5) dans un milieu à une densité de cellules de 50 à 1 000 cellules/cm² pour cultiver les cellules aux passages 2 à 8,
le milieu de culture dans l'étape 5) et 6) comprenant en outre un antioxydant.

2. Composition pour une utilisation selon la revendication 1, la sous-culture de l'étape 5) ou 6) étant réalisée par inoculation des cellules souches monoclonales dans un milieu à une densité de cellules de 1 000 cellules/cm².

3. Composition pour une utilisation selon la revendication 1, la pancréatite étant une pancréatite chronique ou une pancréatite aiguë.

4. Composition pour une utilisation selon la revendication 1, les cellules souches monoclonales améliorant au moins une affection pathologique liée à une pancréatite choisie dans le groupe constitué par un œdème, une nécrose, une hémorragie et une infiltration inflammatoire.
